# EUROPEAN PATENT APPLICATION

(11) **EP 4 803 088 A1**
(43) Date of publication of application: **09.09.2026**
(21) Application number: 24884954.9
(22) Date of filing: 01.11.2024
(51) Int. Cl.: A61K 31/7088, C12N 15/115, A61P 19/02, A61K 31/711

(54) **APTAMER CONJUGATE FOR TARGETED DEGRADATION OF SCLEROSTIN**

(30) Priority: 01.11.2023 WO PCT/CN2023/128951
(71) Applicant: Aptacure Therapeutics Limited, Hong Kong (HK)
(72) Inventor: HE, Yixin, Hong Kong Science Park, No. 15 Science Park West Avenue, Pak Shek Kok, New Territories Hong Kong (CN)
(74) Representative: Nederlandsch Octrooibureau
(86) International application number: PCT/CN2024/129258
(87) International publication number: WO 2025/092956

(57) **Abstract**

The present invention relates to the field of biomedicine. Specifically, the present invention relates to an aptamer conjugate for targeted degrading sclerostin. More particularly, the present invention relates to a conjugate of an aptamer against sclerostin and a ligand that can target protein degradation pathways, and the use of said conjugate in degrading sclerostin.

## Description

### Technical Field

The present invention relates to the field of biomedicine. Specifically, the present invention relates to an aptamer conjugate for targeted degradation of Sclerostin. More specifically, the present invention relates to a conjugate of an aptamer against sclerostin and a ligand capable of targeting the protein degradation pathway, as well as the use of said conjugate for degrading sclerostin.

### BACKGROUND OF THE INVENTION

At present, targeted protein degradation (TPD) has become a promising targeted therapy technology, which mainly relies on two protein degradation pathways: proteasome pathway and lysosome pathway. For the proteasome pathway, the bifunctional molecule that can link a target protein to E3 enzyme is called as proteolysis targeting chimera (PROTAC), which degrades the target protein within the cell by using the ubiquitin-proteasome system through a series of enzymes. For the lysosomal pathway, a well-known bifunctional molecule that can link a target protein to a transmembrane receptor is LYTAC (lysosome-targeting chimera), which mediates endocytosis of the target protein into lysosome after endocytosis. Then, the lysosomal microenvironment degrades the target transmembrane or extracellular protein, which is a good complement of PROTAC.

Nucleic acid aptamers are short fragments of DNA or RNA that can recognize and bind to a target molecule (usually a protein) through specific three-dimensional structures. These fragments are selected from a random library of synthesized oligodeoxyribonucleotides or oligonucleotides using SELEX (Systematic Evolution of Ligands by EXponential enrichment). Compared to small molecule ligands, aptamers often have better selectivity and higher affinity. Compared with antibodies, aptamers have simpler evolution processes, are easier to be modified and adjusted in affinity, have lower immunogenicity, and can be designed and engineered universally. Therefore, aptamers become ideal recognition ligands for their targets. Therefore, more and more aptamers have been developed for use as therapeutic agents and probes.

Sclerostin is a promising target for the development of osteoporosis drugs (Rey and Ellies, 2010). It is reported that the humanized monoclonal antibody against human sclerostin promotes bone formation and increases bone mass in clinical trials, with good tolerance. However, therapeutic antibodies have several major problems including high immunogenicity (Padhi, Jang et al., 2011; Padhi, Allison et al., 2014), high production costs (Baker, 2015; Bradbury and Pluckthun 2015; Groff, Brown et al., 2015), instability, requiring continuous cold chain transport and storage (Jayasena, 1999). In addition, the sclerostin antibody may not be able to act on intracellular sclerostin. Thus, for therapies targeting sclerostin, there is a need for alternative sclerostin inhibitors that are non-immunogenic, easy to produce at low cost, highly stable and capable of targeting intracellular sclerostin.

### SUMMARY OF THE INVENTION

The present disclosure provides at least the following embodiments:
Embodiment 1. An aptamer conjugate comprising i) an aptamer that specifically binds to sclerostin, and ii) a specific ligand of E3 ubiquitin ligase (E3) and/or a specific ligand of asialoglycoprotein receptor (ASGPR).
Embodiment 2. The aptamer conjugate of Embodiment 1, wherein the aptamer that specifically binds to sclerostin comprises
(i) a nucleotide sequence having at least about 90%, at least about 91%, at least about 92%, at least about 93%, at least about 94%, at least about 95%, at least about 96%, at least about 97%, at least about 98%, or at least about 99% identity with any one of SEQ ID NOs: 1 to 17; or
ii) at least 30, at least 35, at least 40, at least 45, at least 50 or more consecutive nucleotides in any one of SEQ ID NOs: 1 to 17; or
iii) a nucleotide sequence of any one of SEQ ID NOs: 1 to 17, preferably the nucleotide sequence of SEQ ID NO: 17.

| SEQ ID NO | Name | Sequence | K_{d} (nM) |
|---|---|---|---|
| 1 | aptscl132 | CCCAGACGAGACACCTCATGCTTTTCCCCGGGGGAGGGGT AT | 42.2 |
| 2 | aptscl6 | CGTACGGTCGACGCTAGCTGGAAGGGTGGGGGCGGGGGG TCCTCGCCTCGAACGTACGCACGTGGAGCTCGGATCC | 4.2 |
| 3 | aptscl9 | CGTACGGTCGACGCTAGCTGGGGGTAGGGGGACCCTGGCT AGTTAGTCACCGTTTCGACACGTGGAGCTCGGATCC | 3.4 |
| 4 | aptscl15 | CGTACGGTCGACGCTAGCGAGGGGGCCAACTATGCTTAGT GGGGGGGTTGACCGTATCCACGTGGAGCTCGGATCC | 4 |
| 5 | aptscl46 | CGTACGGTCGACGCTAGCGGGTGGATTAAGGGGGCCCCGT CTGTAGGCGATTGGCGAACACGTGGAGCTCGGATCC | 45.6 |
| 6 | aptscl51 | CGTACGGTCGACGCTAGCTGGGGGTAGGGGGTCCTTGGCT ACGGTGATTCGGATGTGACACGTGGAGCTCGGATCC | 62.2 |
| 7 | aptscl1 | CGTACGGTCGACGCTAGCTCTTGTTCATCGATCCTACGCAC GTGGAGCTCGGATCC | 0.02 |
| 8 | aptscl2 | CGTACGGTCGACGCTAGCCAGCCAAAGACAGAGATGCAC GTGGAGCTCGGATCC | 0.006 |
| 9 | aptscl3 | CGTACGGTCGACGCTAGCTGTTGTACATCGCCTTACGCACG TGGAGCTCGGATCC | 0.04 |
| 10 | aptscl5 | CGTACGGTCGACGCTAGCTGTTGTTCATCGACTTGACGCA CGTGGAGCTCGGATCC | 0.005 |
| 11 | aptscl8 | CGTACGGTCGACGCTAGCCGGCGAAATTGCTACCACGTGG AGCTCGGATCC | 0.005 |
| 12 | aptscl12 | CGTACGGTCGACGCTAGCCGTTGACTCGTTGCTACACGTG GAGCTCGGATCC | 0.005 |
| 13 | aptscl16 | CGTACGGTCGACGCTAGCTGGTTTCATCGATCGTATCCACG TGGAGCTCGGATCC | 0.61 |
| 14 | aptscl22 | CGTACGGTCGACGCTAGCTGGGTTTATCGACTAGTTCCACG TGGAGCTCGGATCC | 0.76 |
| 15 | aptscl29 | CGTACGGTCGACGCTAGCCGGAGACCTGAGGTTCACGTGG AGCTCGGATCC | 0.28 |
| 16 | aptscl32 | CGTACGGTCGACGCTAGCTGTGTTAATCGCCGTACCTCCAC GTGGAGCTCGGATCC | 0.18 |
| 17 | aptscl56 | CGGGGTGTGGGTTCGTCGTTAGCTTGATTTGGCAGCTGCC | 45 |

Embodiment 3. The aptamer conjugate of Embodiment 1 or 2, wherein the aptamer that specifically binds to sclerostin has a Kd (dissociation constant) of less than 100 nM, preferably less than 50 nM, more preferably less than 40 nM, more preferably less than 30 nM, more preferably less than 20 nM, more preferably less than 10 nM, or less.
Embodiment 4. The aptamer conjugate of any one of Embodiments 1 to 3, wherein the aptamer that specifically binds to sclerostin is a modified aptamer that can comprise one or more modifications conferring enhanced nuclease resistance to said aptamer and/or extending the in vivo half-life of said aptamer.
Embodiment 5. The aptamer conjugate of Embodiment 4, wherein the modification comprises a 3' inverted deoxythymidine (3' idT) modification.
Embodiment 6. The aptamer conjugate of Embodiment 4, wherein the modification comprises replacing one or more naturally occurring nucleotides with a modified nucleotide selected from a 2'-fluoro-, 2'-methoxyethyl-, 2'-methoxy- and/or 2'-allyloxy-modified nucleotide, preferably a 2'-methoxy-modified nucleotide.
Embodiment 7. The aptamer conjugate of Embodiment 4, wherein the modification comprises an inter-nucleotide modification, for example, an inter-nucleotide phosphorothioate bond modification.
Embodiment 8. The aptamer conjugate of Embodiment 4, wherein the aptamer comprises a 2'-methoxy (2'-OMe) modification and/or a 3' inverted deoxythymidine (3'idT) modification.
Embodiment 9. The aptamer conjugate of any one of Embodiments 1 to 8, wherein the aptamer is further conjugated with a fatty acid.
Embodiment 10. The aptamer conjugate of Embodiment 9, wherein the fatty acid is selected from palmitic acid (PA), dodecanedioic acid (DA), tetradecanedioic acid, hexadecanedioic acid, stearic acid (SA), octadecanedioic acid, lauric acid, eicosapentaenoic acid (EPA), docosahexaenoic acid (DHA), and arachidonic acid (ARA), preferably, the fatty acid is octadecanedioic acid.
Embodiment 11. The aptamer conjugate of any one of Embodiments 1 to 10, wherein the specific ligand of E3 is selected from von Hippel-Landau (VHL), murine double minute 2 (MDM2) and cereblon (CRBN); preferably, the specific ligand of E3 is VHL, for example, the VHL comprises a structure shown in the following formula: 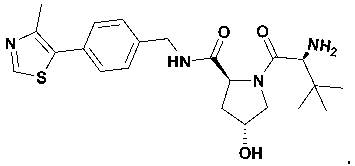
Embodiment 12. The aptamer conjugate of any one of Embodiments 1 to 11, wherein the specific ligand of E3 is conjugated with the aptamer via a linker, for example, conjugated to the 5' end of the aptamer.
Embodiment 13. The aptamer conjugate of Embodiment 12, wherein the linker is a bifunctional linker,
for example, the bifunctional linker comprises one of the following structures: 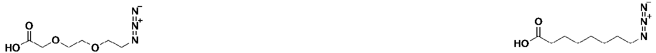 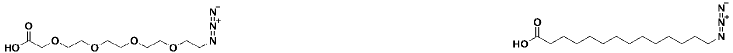 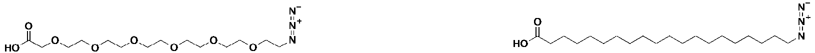 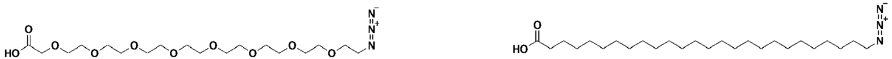 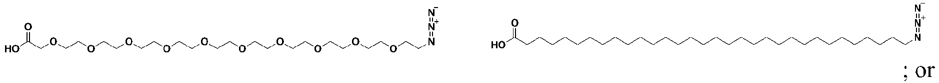
the bifunctional linker comprises the following structure:

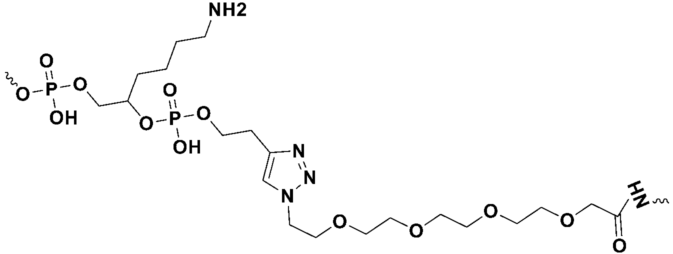

Embodiment 14. The aptamer conjugate of Embodiment 13, wherein the aptamer conjugate comprises a structure of: (FA/E3 ligand)-bifunctional linker-aptamer nucleotide sequence (5'-3'), wherein FA represents a fatty acid and E3 ligand represents the specific ligand of E3, wherein the FA and the E3 ligand are conjugated to the 5' end of the aptamer nucleotide sequence via the bifunctional linker, preferably, the fatty acid is octadecanedioic acid and/or the specific ligand of E3 is VHL.
Embodiment 15. The aptamer conjugate of Embodiment 14, wherein the aptamer conjugate comprises a structure as shown in the following formula: 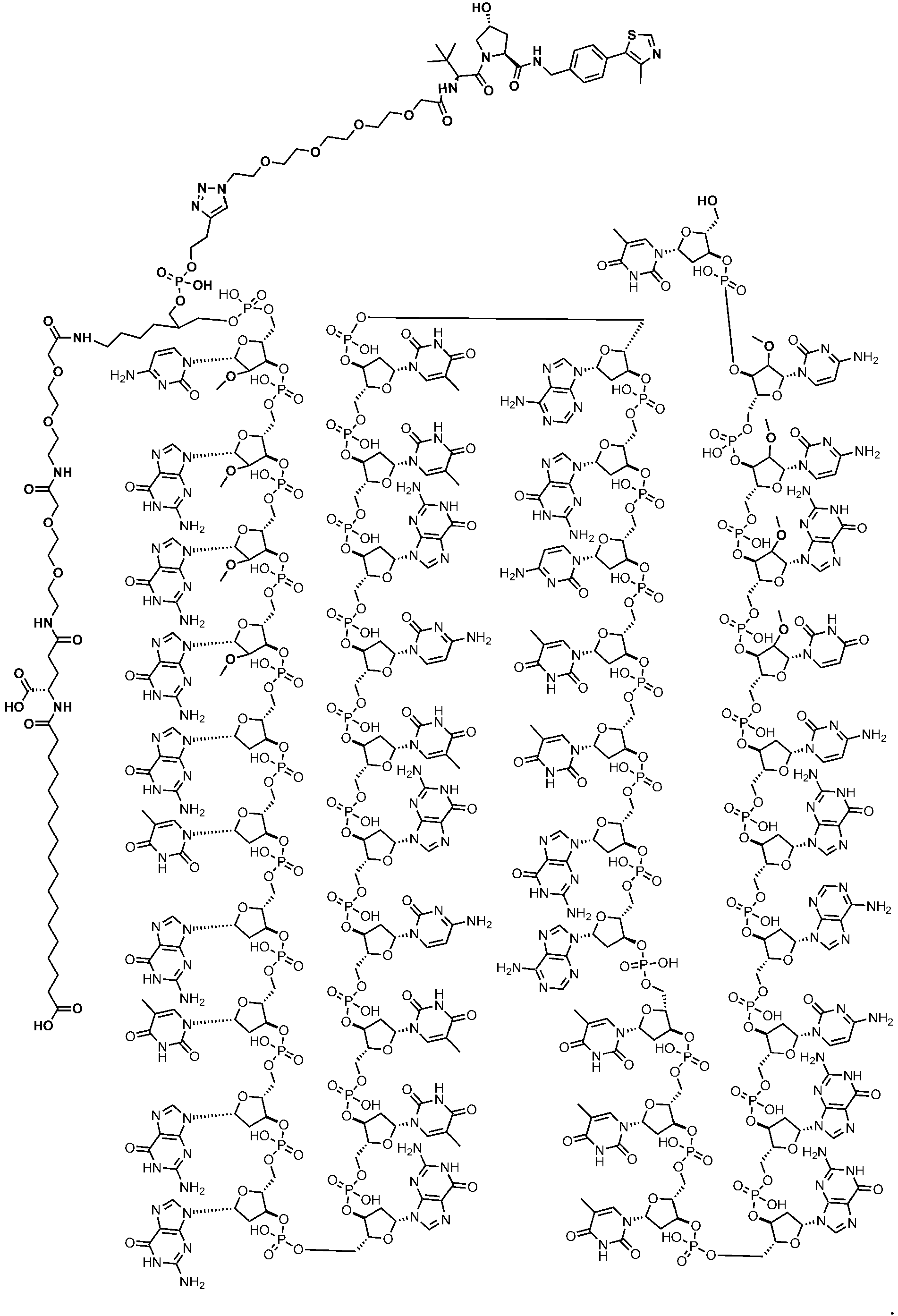
Embodiment 16. The aptamer conjugate of any one of Embodiments 1 to 10, wherein the specific ligand of the asialoglycoprotein receptor (ASGPR) is N-acetylgalactosamine (GalNAc).
Embodiment 17. The aptamer conjugate of any one of Embodiments 1 to 16, wherein the specific ligand of ASGPR such as GalNAc is conjugated to the aptamer via a linker.
Embodiment 18. The aptamer conjugate of Embodiment 16 or 17, wherein the aptamer conjugate comprises a structure shown by the following formula: 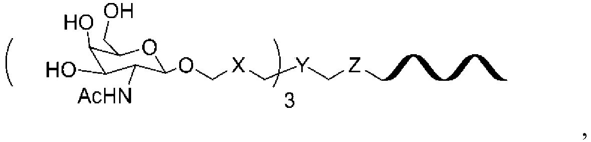 wherein

| | **is selected from** | **preferably,** |
|---|---|---|
| X | 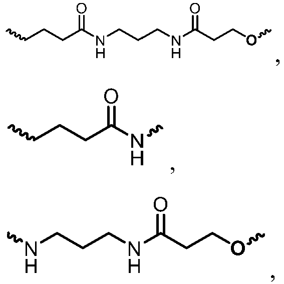 | 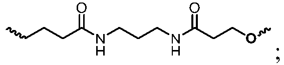 |
| Y | 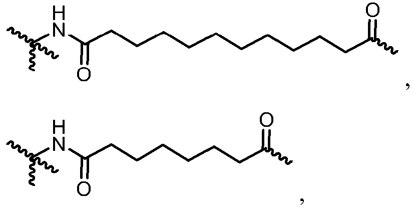 | 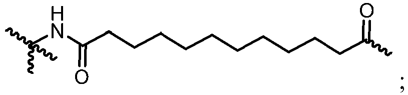 |
| Z | 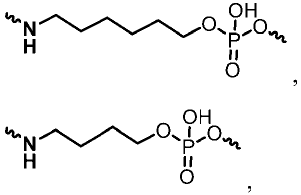 | 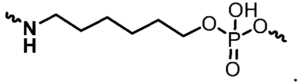 |

Embodiment 19. The aptamer conjugate of any one of Embodiments 16 to 18, wherein the aptamer conjugate comprises a structure shown by the following formula: 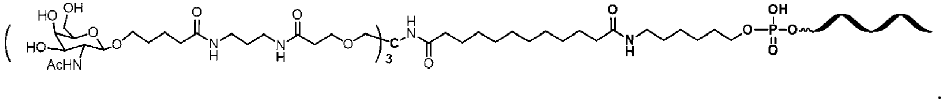
Embodiment 20. The aptamer conjugate of any one of Embodiments 1 to 19, wherein the aptamer nucleotide sequence (in the 5'-3' direction) is:
C(OMe)G(OMe)G(OMe)G(OMe)GTGTGGGTTCGTCGTTAGCTTGATTTGGCAGCU (OMe)G(OMe)C(OMe)C(OMe)-idT, wherein (OMe) represents a 2'-methoxy (2'-OMe) modification of the corresponding nucleotide and idT represents a 3' inverted deoxythymidine modification.
Embodiment 21. The aptamer conjugate of any one of Embodiments 1 to 20, wherein the aptamer conjugate is for targeted degradation of sclerostin.
Embodiment 22. The aptamer conjugate of Embodiment 21, wherein the aptamer conjugate is used for targeted degradation of sclerostin within a cell.
Embodiment 23. The aptamer conjugate of Embodiment 22, wherein the cell is a cancer cell, for example, the cancer cell is a cancer cell highly expressing sclerostin, or the cancer cell is a cancer cell highly expressing sclerostin and asialoglycoprotein receptor (ASGPR).
Embodiment 24. The aptamer conjugate of Embodiment 23, wherein the cancer cell is a breast cancer cell (preferably a triple-negative breast cancer cell) or a liver cancer cell.
Embodiment 25. A method of treating a sclerostin-related disease, the method comprising administering a therapeutically effective amount of the aptamer conjugate of any one of Embodiments 1-20 to a subject in need thereof, wherein said subject is for example a human.
Embodiment 26. The method of Embodiment 25, wherein the sclerostin-related disease is selected from the group consisting of osteoporosis, osteopenia, osteomalacia, osteogenesis imperfecta (OI), ischemic bone necrosis, rheumatoid arthritis, fracture, osteoarthritis, multiple myeloma, hypophosphatemic rickets, hepatocellular carcinoma and triple negative breast cancer.
Embodiment 27. A pharmaceutical composition comprising at least one of the aptamer conjugate according to any one of Embodiments 1 to 20 and a pharmaceutically acceptable carrier or excipient.
Embodiment 28. Use of the aptamer conjugate according to any one of Embodiments 1 to 20 or the pharmaceutical composition according to Embodiment 27 in the preparation of a medicine, wherein said medicine is used for treating a sclerostin-related disease.
Embodiment 29. The use of Embodiment 28, wherein the sclerostin-related disease is selected from osteoporosis, osteopenia, osteomalacia, osteogenesis imperfecta (OI), ischemic bone necrosis, rheumatoid arthritis, fracture, osteoarthritis, multiple myeloma, hypophosphatemic rickets, liver cancer and triple negative breast cancer.

### BRIEF DESCRIPTION OF THE DRAWINGS

FIG. 1. Synthesis of the VHL ligand linked to a sclerostin aptamer conjugated with OA. (A) Synthesis route of VHL E3 ligand linked to a sclerostin aptamer conjugated with OA. (B) Purifying Apc001OA-VHL on HPLC. (C) ESI-MS analysis of Apc001OA-VHL.
FIG. 2. The VHL E3 ligand linked to a sclerostin aptamer conjugated with OA can significantly degrade intracellular sclerostin in MDA-MB-231 cells in vitro. The MDA-MB-231 cells were treated with 0.7 µM Apc001OA-VHL, 1.4 µM Apc001OA-VHL and PBS respectively.
FIG. 3. The VHL E3 ligand linked to a sclerostin aptamer conjugated with OA can degrade intracellular sclerostin in MDA-MB-231 cells in vitro for up to 24 hours. The MDA-MB-231 cells were treated with 0.7 µM of Apc001OA-VHL or PBS, respectively.
FIG. 4. The VHL E3 ligand linked to a sclerostin aptamer conjugated with OA can inhibit the proliferation of MDA-MB-231 cells in vitro. The MDA-MB-231 cells were treated with 700 nM of Apc001OA-VHL, Apc001OA or PBS, respectively.
FIG. 5. The VHL E3 ligand linked to a sclerostin aptamer conjugated with OA can inhibit cell migration of MDA-MB-231 cells in vitro. The MDA-MB-231 cells were treated with 700 nM of Apc001OA-VHL, Apc001OA or PBS, respectively.
FIG. 6. The VHL E3 ligand linked to a sclerostin aptamer conjugated with OA can inhibit the cell viability of MDA-MB-231 cells in vitro. The MDA-MB-231 cells were treated with 700 nM Apc001OA-VHL, Apc001OA or PBS, respectively. The statistical results were presented in the form of mean±SD and unpaired t-test.
FIG. 7. The VHL E3 ligand linked to a sclerostin aptamer conjugated with OA can inhibit tumor growth in a subcutaneous mouse model inoculated with MDA-MB-231 cells, and tumor metastasis in an orthotopic mouse model inoculated with 4T1 cells.
FIG. 8. Construction of the GalNAc-Apc001 conjugate.
FIG. 9. GalNAc mediated cell-specific internalization and transport of FAM-labeled Apc001 and scramble sequences to ASGPR+ cell lysosomes. FIG. 6 (a-b): Confocal microscope images of HepG2 cells (a) and A375 cells (b) incubated with 1 µM GalNAc-Apc001-FAM, GalNAc-Scramble-FAM and Apc001-FAM for 2 hours respectively. The cell nucleuses were stained with Hoechst 33342 (blue); FAM (green); and lysosomes were stained with Lysotracker Red. Scale bar, 10 µm.
FIG. 10. GalNAc-Apc001 mediated sclerostin degradation. Western blot was used to analyze the accumulation (0-8 hours) and degradation (8-18 hours) of sclerostin transported by GalNAc-Apc001 into HepG2 cells. HepG2 cells were incubated with DMEM medium containing 1 µM of a GalNAc-Apc001/Sclerositin (1:1) mixture for 0 to 8 hours. At 8 hours, the culture medium was replaced with a normal DMEM culture medium. GAPDH was used as an internal control.

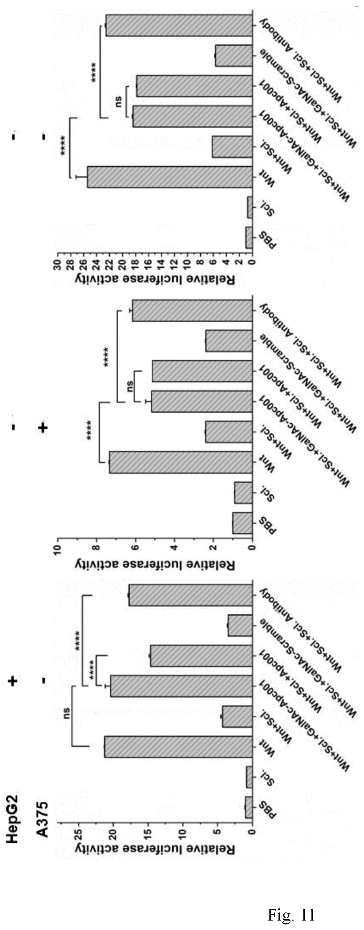

FIG. 11. GalNAc-Apc001 can greatly reduce the sclerostin inhibitory effect on Wnt signaling in vitro with additional ASGPR+ cells. The effect of GalNAc-Apc001, Apc001 and sclerostin antibody (Scl. Antibody) on the inhibition of Wnt signaling induced by sclerostin in HEK 293 cells was tested in the presence or absence of HepG2 cells or A375 cells. One-way variance analysis using Tukey test was used to determine inter-group differences. Each group n=3, *P<0.05, **P<0.01, ***P<0.001. Note: PBS represents the group treated with 1×PBS, which has the same volume as other groups. Scl. represents the group treated with 100 nM sclerostin. Wnt represents the group transfected with Wnt-1 plasmid.

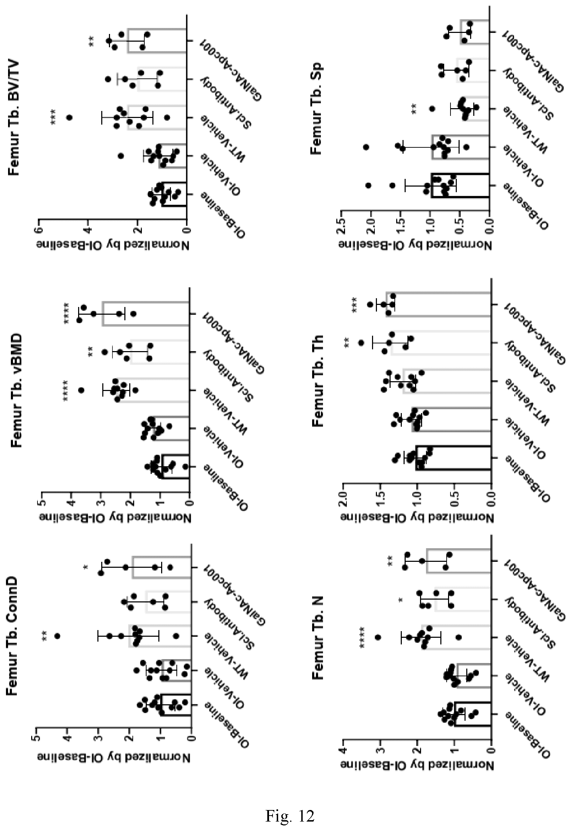

FIG. 12. GalNAc conjugation facilitated the modified aptamer to promote bone anabolism in Osteogenesis Imperfecta mice (Col1a2+/G610C). Bar graphs of structural parameters TB.conn.D, TB.vBMD, TB.BV/TV, TB.N, TB.Th and TB.Sp from ex vivo micro-CT examination of trabecular bone in distal femur. Note: TB.conn.D: trabecular bone connectivity; TB.vBMD: trabecular volumetric mineral density; TB.BV/TV: trabecular volume fraction; TB.N: number of trabeculae; TB.Th: trabecular thickness; TB.Sp: trabecular separation. Data were standardized by parameters in the OI baseline groups and expressed as mean±SD, followed by one-way ANOVA using Tukey post-hoc test with n=6-12 per group. * P<0.05; ** P<0.01; *** P<0.001; **** compared with OI-Veh, P<0.0001.

### DETAILED DESCRIPTION OF THE INVENTION

Unless otherwise indicated or defined, all terms used herein have their ordinary meanings in the art and will be understood by those skilled in the art. Reference is made to standard manuals such as Sambrook et al., "Molecular Cloning: A Laboratory Manual"; Lewin, "Genes VIII"; and Roitt et al., "Immunology" (8th ed.), and the general prior art cited herein; in addition, all methods, steps, techniques, and procedures not specifically described are performed and have been performed in a manner known to those skilled in the art. Also, reference is made to, for example, standard handbooks, the above general prior art and other references cited therein.

### Definitions

As used herein, the term "nucleotide" refers to a ribonucleotide or a deoxyribonucleotide, or a modified form thereof, as well as an analog thereof. Nucleotides include species that include purines (e.g., adenine, hypoxanthine, guanine, and their derivatives and analogs) as well as pyrimidines (e.g., cytosine, uracil, thymine, and their derivatives and analogs).

As used herein, "nucleic acid," "oligonucleotide," and "polynucleotide" are used interchangeably to refer to a polymer of nucleotides and include DNA, RNA, DNA/RNA hybrids and modifications of these kinds of nucleic acids, oligonucleotides and polynucleotides, wherein the attachment of various entities or moieties to the nucleotide units at any position are included. The terms "polynucleotide," "oligonucleotide," and "nucleic acid" include double- or single- stranded molecules. Nucleic acid, oligonucleotide, and polynucleotide are broader terms than the term aptamer and, thus, the terms nucleic acid, oligonucleotide, and polynucleotide include aptamers but are not limited to aptamers.

As used herein, "aptamer" refers to a non-naturally occurring nucleic acid that has a desirable action on a target molecule. A desirable action includes, but is not limited to, binding of the target, catalytically changing the target, reacting with the target in a way that modifies or alters the target or the functional activity of the target, covalently attaching to the target, and facilitating the reaction between the target and another molecule. In one embodiment, the action is specific binding affinity for a target molecule (such as, sclerostin), such target molecule being a three dimensional chemical structure other than a polynucleotide that binds to the nucleic acid ligand through a mechanism which is independent of Watson/Crick base pairing or triple helix formation, wherein the aptamer is not a nucleic acid having the known physiological function of being bound by the target molecule. In this context, the "specific binding affinity" of an aptamer for its target (such as, sclerostin) means that the aptamer binds to its target generally with a much higher degree of affinity than it binds to other, non-target, components in a mixture or sample. The aptamer may be a single-stranded DNA, a single-stranded RNA, a single-stranded DNA/RNA hybrid or a double-stranded DNA molecule.

Sequence "identity" has an art-recognized meaning and the percentage of sequence identity between two nucleic acid or polypeptide molecules or regions can be calculated using published techniques. Sequence identity can be measured along the full length of a polynucleotide or polypeptide or along a region of the molecule. (See, e.g.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity between two polynucleotide or polypeptides, the term "identity" is well known to skilled artisans (Carrillo, H. & Lipman, D., SIAM J Applied Math 48:1073 (1988)). Many algorithms can be used to determine the percent sequence identity. One example of an algorithm that is suitable for determining percent sequence identity is the algorithm used in the basic local alignment search tool (hereinafter "BLAST"), see, e.g. Altschul et al, J. Mol. Biol. 215:403-410, 1990 and Altschul et al, Nucleic Acids Res., 15:3389-3402, 1997. Software for performing BLAST analyses is publicly available through the National Center for Biotechnology Information (hereinafter "NCBI"). The default parameters used in determining sequence identity using the software available from NCBI, e.g., BLASTN (for nucleotide sequences) are described in McGinnis et al, Nucleic Acids Res., 32: W20-W25, 2004.

### Aptamer conjugate

In one aspect, the invention provides an aptamer conjugate comprising i) an aptamer that specifically binds to sclerostin and ii) a specific ligand of E3 ubiquitin ligase (E3) and/or a specific ligand of asialoglycoprotein receptor (ASGPR).

The sclerostin described herein is preferably human sclerostin.

An exemplary human sclerostin comprises the following amino acid sequence:

QGWQAFKNDATEIIPELGEYPEPPPELENNKTMNRAENGGRPPHHPFETKDVSEY
SCRELHFTRYVTDGPCRSAKPVTELVCSGQCGPARLLPNAIGRGKWWRPSGPDFRCIP
DRYRAQRVQLLCPGGEAPRARKVRLVASCKCKRLTRFHNQSELKDFGTEAARPQKGR
KPRPRARSAKANQAELENAY

In some embodiments, the aptamer comprises a nucleotide sequence having at least about 90%, at least about 91%, at least about 92%, at least about 93%, at least about 94%, at least about 95%, at least about 96%, at least about 97%, at least about 98%, or at least about 99% identity to any one of SEQ ID NOs: 1-17; or the aptamer comprises at least 30, at least 35, at least 40, at least 45, at least 50, or more consecutive nucleotides in any one of SEQ ID NOs: 1-17. In some embodiments, the aptamer specifically binds to sclerostin. In some preferred embodiments, the aptamer comprises a nucleotide sequence of any one of SEQ ID NOs: 1 to 17. More preferably, the aptamer comprises a nucleotide sequence of SEQ ID NO: 17.

In some embodiments, the K_{d} (dissociation constant) of the aptamer or aptamer conjugate of the invention against sclerostin is less than 100 nM, preferably less than 50 nM, more preferably less than 40 nM, more preferably less than 30 nM, more preferably less than 20 nM, more preferably less than 10 nM, or even less. The Kd is, for example, determined by ELONA (Enzyme-Linked Oligonucleotide Assay).

In some embodiments, the aptamer of the present invention may be a modified aptamer which can comprise one or more modifications. For example, the modification is a modification that confers enhanced nuclease resistance to the aptamer and/or a modification that prolongs the in vivo half-life of the aptamer.

The modification includes, for example, 3' and/or 5' modification such as capping at 3' and 5'. In some embodiments, the nucleic acid molecule is capped with an inverted 3'-deoxythymidine at its 3' end, i.e., a 3' inverted deoxythymidine (3' idT) modification.

The modification may also include substitution of one or more naturally occurring nucleotides with modified nucleotides. For example, the modified nucleotides include but are not limited to 2'-fluoro-, 2'-methoxyethyl-, 2'-methoxy- and/or 2'-allyloxy- modified nucleotides (i.e., the 2'-position hydroxyl group of the ribose is substituted by fluorine, methoxyethyl, methoxy or allyloxy, etc.). The modified nucleotide may also include a C-5 modified pyrimidine. The term "C-5 modified pyrimidine" refers to a pyrimidine having modification at the C-5 position. The C-5-modified pyrimidine can enhance the nuclease resistance of an oligonucleotide, and is well known in the art (see, e.g., International Patent Application WO 2011/130195 and references cited therein). In some preferred embodiments, the modification is 2'-methoxy (2'-OMe) modification. In some embodiments, the modification, such as 2'-methoxy (2'-OMe) modification, is performed on one or more nucleotides, for example four nucleotides, at 5' and/or 3' end of a nucleic acid molecule.

The modifications also include internucleotide modifications, such as those internucleotide modifications with uncharged bonds (such as methyl phosphonate, phosphotriester, phosphoamine ester, carbamate, etc.) and those internucleotide modifications with charged bonds (such as phosphorothioate, phosphorodithioate, etc.), internucleotide modifications with intercalating agents (such as acridine, psoralen, etc.), internucleotide modifications containing chelating agents (such as metals, radioactive metals, boron, oxidizing metals, etc.), internucleotide modifications containing alkylating agents, and internucleotide modifications with modified bonds (for example, alpha anomeric nucleic acid, etc.).

In some embodiments, the aptamer may comprise a combination of various modifications described above. For example, the aptamer may include 2'-methoxy (2'-OMe) modification and/or 3' inverted deoxythymidine (3' idT) modification.

In some embodiments, the aptamer may be conjugated to a fatty acid and/or a coumarin derivative. Conjugation of the aptamer molecule with a fatty acid and/or a coumarin derivative can significantly prolong its in vivo half-life.

In some embodiments, the fatty acid includes but not limited to palmitic acid (PA), dodecanedioic acid (DA), tetradecanedioic acid, hexadecanedioic acid, stearic acid (SA), octadecanedioic acid, lauric acid, eicosapentaenoic acid (EPA), docosahexaenoic acid (DHA), arachidonic acid (ARA) and the like. In some preferred embodiments, the fatty acid is octadecanedioic acid.

In some embodiments, the coumarin derivative includes but is not limited to 4-hydroxycoumarin, 3-acetyl-6-carboxylcoumarin, warfarin, (2-oxo-2H-chromene-3-yl)acetic acid, [(8-acetyl-4-methyl-2-oxo-2H-chromene-7-yl)oxy]acetic acid, coumarin-3-carboxylic acid, N-(4-methyl-7-coumarin) oxamide, 7-(carbomethyl)-4-methylcoumarin, 7-methoxy coumarin-3-carboxylic acid or 6-methoxy-2-oxo-2H-chromene-3-carboxylic acid. In some preferred embodiments, the coumarin derivative is 4-hydroxycoumarin.

In some embodiments, the fatty acid, for example, octadecanedioic acid is conjugated to the 5' end of the aptamer. In some embodiments, the coumarin derivative such as 4-hydroxycoumarin is conjugated to the 5' end of the aptamer. In some embodiments, the fatty acid such as octadecanedioic acid and the coumarin derivative such as 4-hydroxycoumarin are conjugated to the 5' end of the aptamer.

In some embodiments, the fatty acid is conjugated to the aptamer via a linker. In some embodiments, the coumarin derivative such as 4-hydroxycoumarin is conjugated to the aptamer via a linker. In some embodiments, the fatty acid such as octadecanedioic acid and the coumarin derivative such as 4-hydroxycoumarin are conjugated to the aptamer via a linker.

In some embodiments, the E3-specific ligand includes but is not limited to von Hippel-Landau (VHL), murine double minute 2 (MDM2) and cereblon (CRBN). In some preferred embodiments, the E3-specific ligand is VHL. For example, the VHL comprises a structure shown in the following formula:

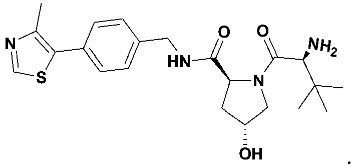

In some embodiments, the E3-specific ligand is conjugated to the aptamer via a linker. In some embodiments, the linker is a bifunctional linker. The bifunctional linker may allow the aptamer to be conjugated with two different molecules, for example, with a fatty acid and an E3-specific ligand.

In some embodiments, the bifunctional linker comprises one of the following structures:

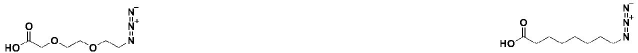

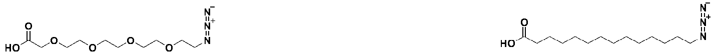

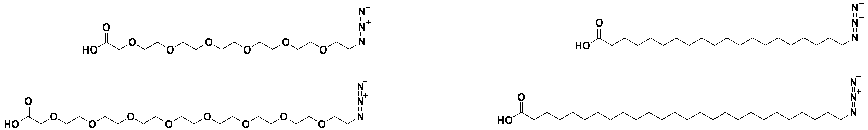

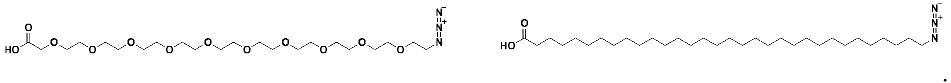

In some embodiments, the linker comprises:

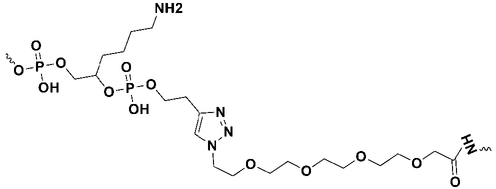

In some embodiments, the aptamer conjugate comprises a structure of: (FA/E3 ligand)-bifunctional linker-aptamer nucleotide sequence (5'-3'), wherein FA represents a fatty acid and E3 ligand represents an E3-specific ligand, wherein FA and E3 ligand are conjugated to 5' end of the aptamer nucleotide sequence via a bifunctional linker.

In some preferred embodiments, the fatty acid is octadecanedioic acid.

In some specific embodiments, the aptamer conjugate comprises a structure as shown in the following formula:

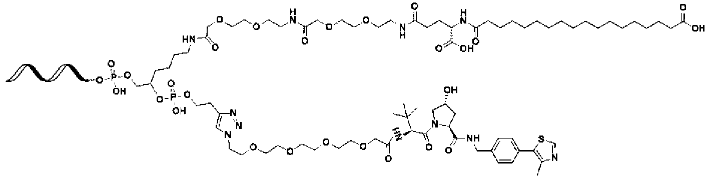

In the structural formula of the invention, the following structure represents a nucleotide sequence (5'-3' direction) of an aptamer (modified or unmodified):

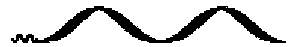

In some specific embodiments, the aptamer conjugate comprises a structure shown by the following formula:

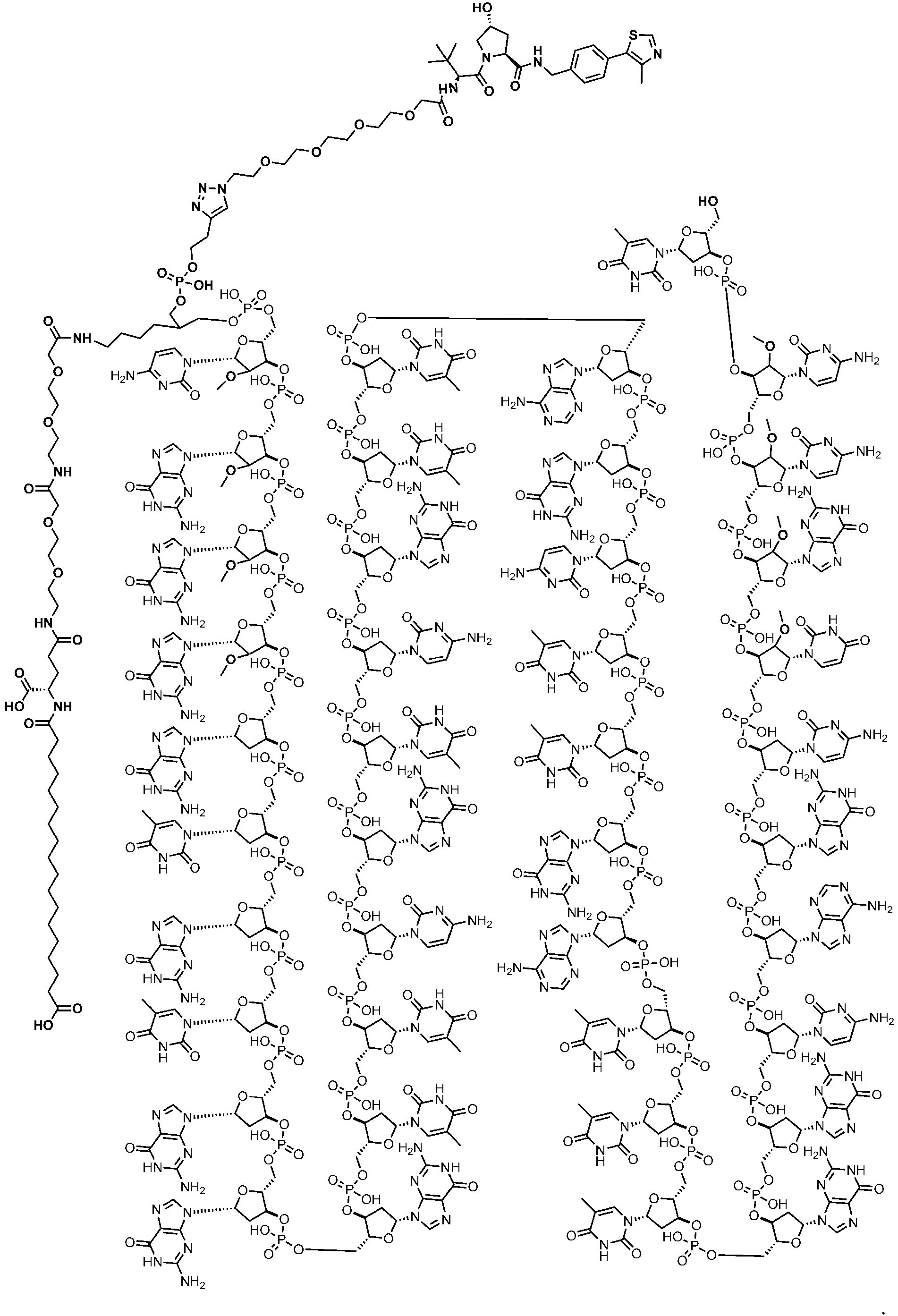

In some embodiments, the ASGPR-specific ligand is N-acetylgalactosamine (GalNAc). In some embodiments, the ASGPR-specific ligand such as GalNAc is conjugated to the aptamer via a linker.

In some embodiments, the aptamer conjugate comprises a structure as shown in the following formula: 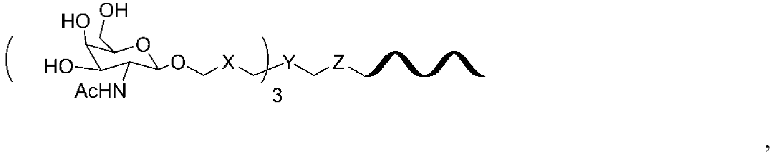 wherein

| | | |
|---|---|---|
| | is selected from | preferably, |
| X | 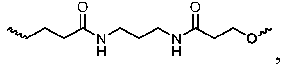 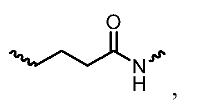 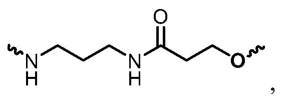 | 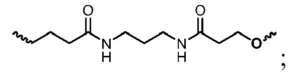 |
| Y | 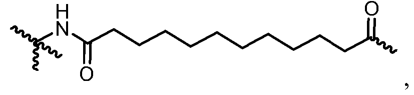 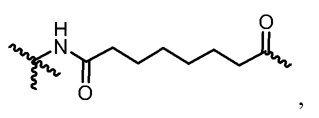 | 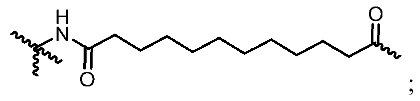 |
| Z | 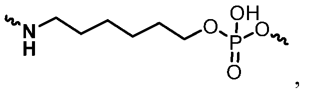 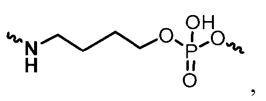 | 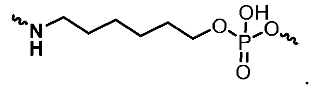 |

In some embodiments, the aptamer conjugate comprises a structure as shown in the following formula:

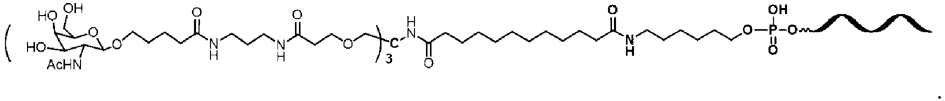

In some preferred embodiments of various aspects of the present invention, the aptamer nucleotide sequence (5'-3' direction) is C(OMe)G(OMe)G(OMe)G(OMe)GTGTGGGTTCGTCGTTAGCTTGATTTGGCAGCU(OM e)G(OMe)C(OMe)C(OMe)-idT, wherein (OMe) represents 2'-methoxy (2'-OMe) modification of the corresponding nucleotide and idT represents a 3' invented deoxythymidine modification.

In some embodiments, the aptamer or aptamer conjugate of the invention inhibits the biological activity of sclerostin. "Inhibition" means that the biological activity of sclerostin is reduced in the presence of the aptamer or aptamer conjugate compared with the absence of the aptamer or aptamer conjugate, for example, reduced by at least about 10%, at least about 20%, at least about 30%, at least about 40%, at least about 50%, at least about 60%, at least about 70%, at least about 80%, even at least about 90%.

As used herein, the term "biological activity" refers to an effect on one or more cellular or extracellular processes that may affect a physiological or pathological process. The biological activity of sclerostin includes, but is not limited to, antagonizing Wnt signaling pathway.

In some embodiments, the aptamer or aptamer conjugate of the present invention can inhibit the antagonistic effect of sclerostin on Wnt signaling pathway. For example, the aptamer or aptamer conjugate of the present invention can block the antagonistic effect of sclerostin in a cell-based Wnt signaling assay.

In some embodiments, the aptamer or the aptamer conjugate of the present invention inhibits biological activity of sclerostin, for example antagonistic effect of sclerostin on Wnt signaling pathway with an EC₅₀ value less than 100 µg/ml, preferably less than 50 µg/ml, more preferably less than 40 µg/ml, more preferably less than 30 µg/ml, more preferably less than 20 µg/ml, more preferably less than 10 µg/ml or even less. In some embodiments, the EC₅₀ value was determined in vitro by TOP-Wnt-induced luciferase reporter gene assay in osteoblasts.

In some embodiments, the aptamer conjugate of the invention is used for targeted degrading sclerostin.

In some embodiments, the aptamer conjugate is used for targeted degrading sclerostin within a cell.

In some embodiments, the cell is a cancer cell. In some embodiments, the cancer cell is a cancer cell highly expressing sclerostin. In some embodiments, the cancer cell is a cancer cell highly expressing sclerostin and asialoglycoprotein receptor (ASGPR). In some preferred embodiments, the cancer cell is a breast cancer cell (preferably triple-negative breast cancer cell) or liver cancer cell.

### Treatment of diseases

In another aspect, the invention provides a method of treating a disease by the aptamer conjugate of the invention, comprising administering a therapeutically effective amount of the aptamer conjugate of the invention to a subject in need thereof.

Diseases treated by the aptamer conjugate of this invention include sclerostin-related diseases, such as sclerostin-mediated diseases. In some embodiments, the disease is a disease caused by high expression of sclerostin.

As used herein, a "sclerostin-related disease" includes conditions in which bone mineral density (BMD) is abnormal and/or pathologically low relative to a healthy subject. Diseases characterized by low BMD and/or bone brittleness include, but are not limited to: primary and secondary osteoporosis, osteopenia, osteomalacia, osteogenesis imperfecta (OI), ischemic necrosis (bone necrosis), fracture and implant healing (dental implant and hip implant), bone loss due to other conditions (e.g., associated with HIV infection, cancer and arthritis). Other "sclerostin-related diseases" include, but are not limited to: hypophosphatemic rickets, rheumatoid arthritis, osteoarthritis, arthritis and osteolytic lesions.

As used herein, a "sclerostin-related disease" includes sclerostin-related cancer such as a cancer with high expression of sclerostin. Examples of the cancers include, but are not limited to, myeloma (e.g., multiple myeloma with lytic lesions), breast cancer (e.g., triple-negative breast cancer), colon cancer, melanoma, liver cancer (hepatocellular carcinoma), epithelial cancer, esophageal cancer, brain cancer, lung cancer, prostate cancer or pancreatic cancer, and any metastases thereof. In some embodiments, the cancer is breast cancer, preferably triple negative breast cancer. In some embodiments, the cancer is liver cancer.

The term "sclerostin-related disease" may also include renal and cardiovascular diseases caused by the expression of sclerostin in kidney and cardiovascular system. Said diseases include but are not limited to such renal disorders as glomerular diseases (e.g., acute and chronic glomerulonephritis, rapidly progressive glomerulonephritis, nephrotic syndrome, focal proliferative glomerulonephritis, glomerular lesions associated with systemic disease, such as systemic lupus erythematosus, Goodpasture's syndrome, multiple myeloma, diabetes, polycystic kidney disease, neoplasia, sickle cell disease, and chronic inflammatory diseases), tubular diseases (e.g., acute tubular necrosis and acute renal failure, polycystic renal disease, medullary sponge kidney, medullary cystic disease, nephrogenic diabetes, and renal tubular acidosis), tubulointerstitial diseases (e.g., pyelonephritis, drug and toxin induced tubulointerstitial nephritis, hypercalcemic nephropathy, and hypokalemic nephropathy) acute and rapidly progressive renal failure, chronic renal failure, nephrolithiasis, gout, vascular diseases (e.g., hypertension and nephrosclerosis, microangiopathic hemolytic anemia, atheroembolic renal disease, diffuse cortical necrosis, and renal infarcts), or tumors (e.g., renal cell carcinoma and nephroblastoma).

Said sclerostin-related diseases also include but are not limited to such cardiovascular disorders as ischemic heart disease (e.g., angina pectoris, myocardial infarction, and chronic ischemic heart disease), hypertensive heart disease, pulmonary heart disease, valvular heart disease (e.g., rheumatic fever and rheumatic heart disease, endocarditis, mitral valve prolapse, and aortic valve stenosis), congenital heart disease (e.g., valvular and vascular obstructive lesions, atrial or ventricular septal defect, and patent ductus arteriosus), or myocardial disease (e.g., myocarditis, congestive cardiomyopathy, and hypertrophic cariomyopathy).

The subject may be any animal (domesticated, livestock or wild), including but not limited to cats, dogs, horses, pigs and cows, and preferably human subjects. As used herein, the terms "patient", "individual", and "subject" may be used interchangeably.

The subject may be male or female. Preferably, the human subject is at risk of fracture and more preferably the human subject is at risk of osteoporosis or suffers from osteoporosis. The human subject is preferably a female, and more preferably a female at risk for postmenopausal osteoporosis or suffering from postmenopausal osteoporosis. It is expected that the method of the present invention will be beneficial to a subject at any stage of osteoporosis.

As used herein, "treating" a subject suffering from a disease means that symptoms of the subject are partially or completely alleviated or remain unchanged after treatment. Thus, treatment includes prevention, treatment and/or cure. Prevention refers to prevention of a potential disease and/or prevention of worsening of symptoms or disease progression.

As used herein, "therapeutically effective amount" or "therapeutically effective dose" refers to an amount of a substance, compound, material, or composition comprising a compound that is at least sufficient to produce a therapeutic effect after administration to a subject. Thus, it is the amount necessary to prevent, cure, ameliorate, arrest or partially arrest the symptoms of the disease or condition. As used herein, "therapeutic effect" means an effect resulting from treatment of an individual that alters, generally ameliorates or alleviates the symptoms of the disease or disease condition, or cures the disease or disease condition.

The dosage regimen of the aptamer conjugate is selected according to a variety of factors, including, for example, the type, species, age, weight, gender, and medical condition of the patient; the severity of the condition to be treated; the route of administration; the kidney function and liver function of the patient; and the specific aptamer conjugate or salt thereof as used. An ordinary skilled physician can easily determine and specify the effective amount of the composition required to prevent, combat, or inhibit the progression of the condition.

Generally, the dosing regimen of the aptamer conjugate is about 1 µg/kg body weight to about 100 mg/kg body weight per day.

An exemplary treatment regime entails administration once daily, once every two days, once per week, twice per week, once every two weeks, once every three weeks, once every four weeks, once a month, once every 3 months or once every three to 6 months, or with a short administration interval at the beginning (such as once per week to once every three weeks), and then an extended interval later (such as once a month to once every three to 6 months). The frequency and interval of administration can be determined by those skilled in the art according to the pharmacokinetic parameters of the aptamer conjugate.

### Pharmaceutical Composition

In another aspect, the present invention also provides a pharmaceutical composition comprising at least one at least one aptamer conjugate of the present invention and a pharmaceutically acceptable carrier or excipient. The pharmaceutical composition is, for example, used to treat a sclerostin-related disease.

The aptamer conjugate described herein can be utilized in any pharmaceutically acceptable dosage form, including but not limited to injectable dosage forms, liquid dispersions, gels, aerosols, ointments, creams, lyophilized formulations, dry powders, tablets, capsules, controlled release formulations, fast melt formulations, delayed release formulations, extended-release formulations, pulsatile release formulations, mixed immediate release and controlled release formulations, etc. Specifically, the aptamer conjugate described herein can be formulated: (a) for administration selected from any of oral, pulmonary, intravenous, intraarterial, intrathecal, intraarticular, rectal, ophthalmic, colonic, parenteral, intracisternal, intravaginal, intraperitoneal, local, buccal, nasal, and topical administration; (b) into a dosage form selected from any of liquid dispersions, gels, aerosols, ointments, creams, tablets, sachets and capsules; (c) into a dosage form selected from any of lyophilized formulations, dry powders, fast melt formulations, controlled release formulations, delayed release formulations, extended release formulations, pulsatile release formulations, and mixed immediate release and controlled release formulations; or (d) any combination thereof.

Solutions or suspensions used for parenteral, intradermal, or subcutaneous application can comprise one or more of the following components: (1) a sterile diluent such as water for injection, saline solution, fixed oils, polyethylene glycols, glycerin, propylene glycol or other synthetic solvents; (2) antibacterial agents such as benzyl alcohol or methyl parabens; (3) antioxidants such as ascorbic acid or sodium bisulfite; (4) chelating agents such as ethylenediaminetetraacetic acid; (5) buffers such as acetates, citrates or phosphates; and (6) agents for the adjustment of tonicity such as sodium chloride or dextrose. The pH can be adjusted with an acid or a base (such as hydrochloric acid or sodium hydroxide). A parenteral preparation can be enclosed in ampoules, disposable syringes or multiple dose vials made of glass or plastic.

Pharmaceutical compositions suitable for injectable use may include sterile aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). In all cases, the composition should be sterile and should be fluid to the extent that easy syringability exists. The pharmaceutical composition should be stable under the conditions of manufacture and storage and should be preserved against the contaminating action of microorganisms such as bacteria and fungi. The term "stable", as used herein, means remaining in a state or condition that is suitable for administration to a patient.

The carrier may be a solvent or dispersing medium, including for example water, ethanol, polyols (e.g., glycerol, propylene glycol, liquid polyethylene glycols, etc.) and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol or sorbitol, and inorganic salts such as sodium chloride in the composition. Prolonged absorption of the injectable compositions can be brought about by including in the composition an agent which delays absorption, for example, aluminum monostearate and gelatin.

Sterile injectable solutions can be prepared by incorporating the active reagent (e.g., an aptamer conjugate) in the required amount in an appropriate solvent with one or a combination of ingredients enumerated above, as required, followed by filtered sterilization. Generally, dispersions are prepared by incorporating at least one aptamer conjugate into a sterile vehicle that contains a basic dispersion medium and any other required ingredient. In the case of sterile powders for the preparation of sterile injectable solutions, exemplary methods of preparation include vacuum drying and freeze-drying, both of which will yield a powder of the aptamer conjugate plus any additional desired ingredient from a previously sterile-filtered solution thereof.

Oral compositions generally include an inert diluent or an edible carrier. For example, they may be encapsulated in gelatin capsules or compressed into tablets. For purposes of oral administration, the sclerostin-targeting aptamer conjugate may be incorporated with excipients and used in the form of tablets, troches, or capsules. The formulation may include a pharmaceutically compatible binder and/or adjuvant material as part of the composition.

For administration by inhalation, the compounds are delivered in the form of an aerosol spray from a pressured container or dispenser that contains a suitable propellant, e.g., a gas such as carbon dioxide, a nebulized liquid, or a dry powder from a suitable device. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active reagents are formulated into ointments, salves, gels, or creams as generally known in the art. The reagents can also be prepared in the form of suppositories (e.g., with conventional suppository bases such as cocoa butter and other glycerides) or retention enemas for rectal delivery.

In one embodiment, the aptamer conjugate is formulated for topical administration. As used herein, "topical administration" refers to delivery of an aptamer conjugate to an animal by contacting, directly or otherwise, a formulation comprising the aptamer conjugate to all or a portion of the skin (epidermis) of an animal. The term encompasses several routes of administration including, but not limited to, topical and transdermal. A common requirement for these modes of administration is efficient delivery to the target tissue or stratum. In one aspect, topical administration is used as a means to penetrate the epidermis and dermis and ultimately achieve systemic delivery of the aptamer conjugate. In another aspect, topical administration is used as a means to selectively deliver the aptamer conjugate to the epidermis or dermis of an animal, or to specific strata thereof.

For topical administration, the aptamer conjugate may be formulated into pharmaceutically acceptable ointments, creams, lotions, eye ointments, eye drops, ear drops, impregnated dressings, and aerosols, medicated powders, medicated adhesives, foams, and may contain appropriate conventional additives or excipients, including, for example, preservatives or solvents to assist drug penetration, and emollients in ointments, gels, and creams. Such topical formulations may also contain compatible conventional carriers, for example ethanol or oleyl alcohol for lotions. Such carriers may constitute from about 1% to about 98% by weight of the formulation; more usually, such carriers will constitute up to about 80% by weight of the formulation. Specific formulations for the topical delivery of aptamers are described in the art.

In one embodiment, an aptamer conjugate is prepared with a carrier that will protect against rapid elimination from the body. For example, a controlled release formulation can be used, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions can also be used as pharmaceutically acceptable carriers. These can be prepared according to methods known to those skilled in the art.

Additionally, suspensions of the aptamer conjugate may be prepared as appropriate oily injection suspensions. Suitable lipophilic solvents or vehicles include fatty oils, such as sesame oil, or synthetic fatty acid esters, such as ethyl oleate, triglycerides, or liposomes. Nonlipid polycationic amino polymers may also be used for delivery. Optionally, the suspension may also include suitable stabilizers or agents to increase the solubility of the compounds and allow for the preparation of highly concentrated solutions.

In some cases, it may be especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of an aptamer conjugate calculated to produce the desired therapeutic effect in association with the required pharmaceutical carrier. The specification for the dosage unit forms of the aptamer conjugate described herein are dictated by and directly dependent on the unique characteristics of the particular aptamer conjugate and the particular therapeutic effect to be achieved, and the limitations inherent in the art of compounding such an active agent for the treatment of individuals.

Pharmaceutical compositions comprising at least one aptamer conjugate can include one or more pharmaceutical excipients. Examples of such excipients include, but are not limited to, binding agents, filling agents, lubricating agents, suspending agents, sweeteners, flavoring agents, preservatives, buffers, wetting agents, disintegrants, effervescent agents, and other excipients. Such excipients are known in the art. Exemplary excipients include: (1) binding agents which include various celluloses and cross-linked polyvinylpyrrolidone, microcrystalhne cellulose, such as Avicel PH101 and Avicel PH102, silicified microcrystalhne cellulose (ProSolv SMCC^{™}), gum tragacanth and gelatin; (2) filling agents such as various starches, lactose, lactose monohydrate, and lactose anhydrous; (3) disintegrating agents such as alginic acid, Primogel, corn starch, lightly crosslinked polyvinyl pyrrolidone, potato starch, maize starch, and modified starches, croscarmellose sodium, cross-povidone, sodium starch glycolate, and mixtures thereof; (4) lubricants, including agents that act on the flowability of a powder to be compressed, include magnesium stearate, colloidal silicon dioxide, such as Aerosil 200, talc, stearic acid, calcium stearate, and silica gel; (5) glidants such as colloidal silicon dioxide; (6) preservatives, such as potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quaternary compounds such as benzalkonium chloride; (7) diluents such as pharmaceutically acceptable inert fillers, such as microcrystalhne cellulose, lactose, dibasic calcium phosphate, saccharides, and/or mixtures of any of the foregoing; examples of diluents include microcrystalline cellulose, such as Avicel PH101 and Avicel PH102; lactose such as lactose monohydrate, lactose anhydrous, and Pharmatose DCL21; dibasic calcium phosphate such as Emcompress; mannitol; starch; sorbitol; sucrose; and glucose; (8) sweetening agents, including any natural or artificial sweetener, such as sucrose, saccharin sucrose, xylitol, sodium saccharin, cyclamate, aspartame, and acesulfame; (9) flavoring agents, such as peppermint, methyl salicylate, orange flavoring, Magnasweet (trademark of MAFCO), bubble gum flavor, fruit flavors, and the like; and (10) effervescent agents, including effervescent couples such as an organic acid and a carbonate or bicarbonate.

### Examples

The present invention will be further illustrated by way of examples, but the present invention is not limited to the described examples.

### Example 1. E3 ligand VHL linked to a sclerostin aptamer conjugated with OA can significantly degrade intracellular sclerostin in MDA-MB-231 cells in vitro.

The present inventors identified a sclerostin DNA aptamer (referred to as Apc001), which can promote bone formation in vivo (Wang et al., 2022; Yu et al., 2022). Then, the long-acting Apc001 (Apc001OA) was developed by modifying with octadecanedioic acid (PCT/CN2022/082996) (Zhang et al., 2022b). According to the reports, SOST mRNA expression was found in most clinical TNBC (triple-negative breast cancer) tissues but not detected in healthy breast tissues (Hesse et al., 2019). Recently, the inventors' in vitro data showed that octadecanedioic acid can promote internalization of conjugated Apc001 into MDA-MB-231 cells (TNBC cells) in vitro. Surprisingly, Apc001OA can inhibit the proliferation of MDA-MB-231 cells in vitro while sclerostin antibody cannot. This means that the role of sclerostin in cell proliferation of MDA-MB-231 cells is not yet recognized. The inventors of the present invention wish to solve a scientific problem whether sclerostin can be an attractive therapeutic target for treating TNBC. It is necessary to develop tools for targeting and degrading sclerostin in cells of TNBC.

Proteolysis-Targeting Chimera (PROTAC) is a novel therapeutic strategy that has attracted widespread attention from academia and the pharmaceutical industry (Garber, 2022). The PROTAC degrader comprises two ligands connected by a linker, one of which can bind to the target protein (POI), and the other can bind to an E3 ubiquitin ligase. By forming a POI-PROTAC-E3 ligase ternary complex, PROTAC hijacks the ubiquitin-proteasome system (UPS) to degrade POI (Sakamoto et al., 2001).

Therefore, an E3 ligand conjugated to sclerostin aptamer was designed to degrade intracellular sclerostin in MDA-MB-231 cells in vitro. ApC001OA is an ideal sclerostin ligand because it has high binding affinity with sclerostin and can be internalized into MDA-MB-231 cells in vitro. The VHL ligand is an E3 ligand widely used in PROTAC (Cao et al., 2022), which was linked to Apc001OA by using a PEG linker with good solubility.

The purpose of the present example is to compare the difference in expression level of sclerostin between Apc001OA-VHL and PBS-treated MDA-MB-231 cells by western blot analysis.

### Experimental Design

Object of the experiment: To chemically obtain a VHL ligand conjugated to sclerostin aptamer, the 5'-yne Apc001OA aptamer was linked to azide-linked E3 ligand (including VHL ligand) by copper click reaction. After completion of the reaction, the reaction mixture was purified on a Sephadex^{™}G-25 DNA grade column and HPLC. Then, the VHL E3 ligand linked to Apc0010A (Apc001OA-VHL) was obtained and confirmed by ESI-MS.

The MDA-MB-231 cells were seeded at a density of 4×10 ⁵ per well in a 6-well plate and incubated overnight. Then, the cells were incubated with 0.7 µM Apc001OA-VHL, 1.4 µM Apc001OA-VHL and PBS at 37°C, respectively. After 24 hours, the supernatant was removed and protein extract was isolated from cells. Finally, the protein sample was analyzed by western blot.

### Assessment Protocol

Synthesis of the designed VHL E3 ligand linked to OA-conjugated sclerostin aptamer: Apc001OA (1.0 eq), azide-linked E3 ligand (100 eq) and CuSO₄ (50 eq) were transferred into a 1.5 mL plastic reaction tube. Then, NaHCO₃ (5 µL, 200 mM) and CH₃CN/H₂O (100 µL, V_{CH3CN}/V_{H2O}=6%) were added, and the mixture was briefly vortexed. Next, TCEP (3 µL, 20 mM) was added and the mixture was vortexed further. Then, the reaction tube was briefly purged with nitrogen and kept on a shaker at 25°C for 4 hours (Patil et al., 2021). After completion of the reaction, the reaction mixture was purified by Sephadex^{™}G-25 DNA Grade Column. Then, the crude product was further purified on HPLC by gradient method with 0.5 M TEAA and acetonitrile as solvent. Appropriate fractions were collected and then confirmed by ESI-MS analysis.

Western blotting analysis: All protein samples were separated by denaturing 10% SDS-PAGE gel, set at 80 V for 30 min and then 120 V for 1 h. The gel was transferred to a 0.2 µm PVDF membrane (Bio-Rad) by electroblotting, set at 350 A for 60 minutes. All membranes were blocked with 5% blotting grade block agent (Bio-rad) containing 0.1% Tween 20 (TBST) at room temperature for one hour, followed by incubation overnight with the primary antibody at 4°CThe membrane was incubated with the peroxidase-conjugated secondary antibody at room temperature for 1 hour. According to manufacturer's instruction, the bound antibody was visualized by enhanced chemiluminescence system (ECL) (Bio-rad). Sclerostin was detected by anti-sclerostin polyclonal antibody (Abcam, #ab85799). GAPDH was used as a control and detected by anti-GAPDH monoclonal antibody (Santa Cruz, # sc365062). All western blotting images were processed using Image Lab software.

### Results and Discussion

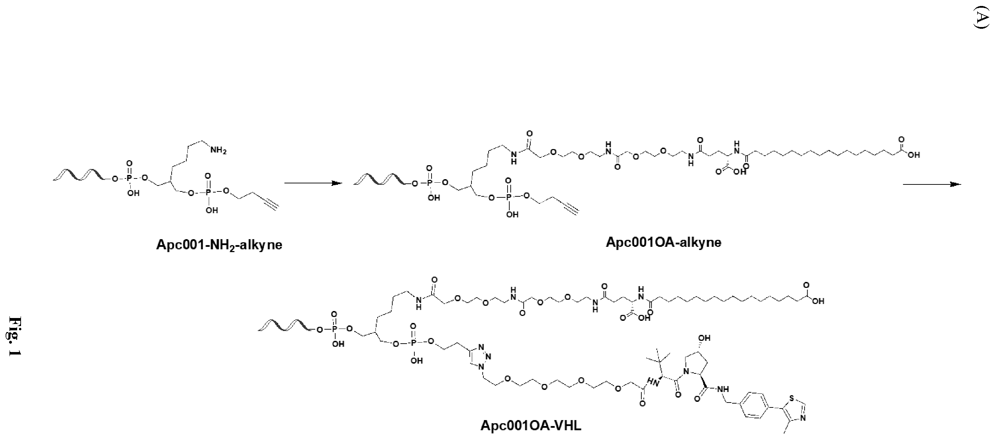

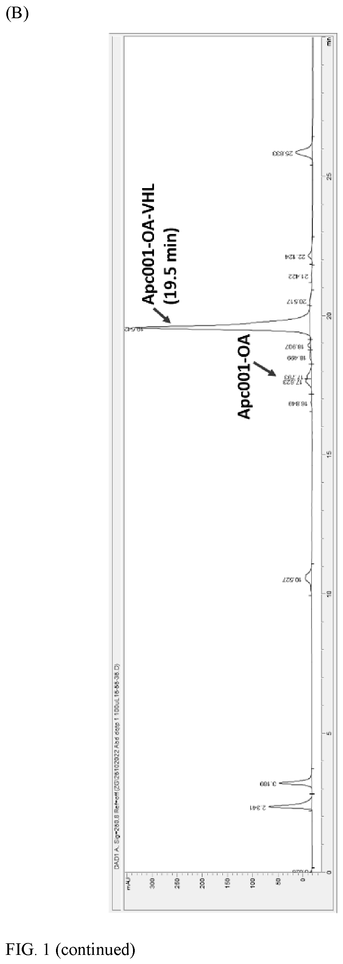

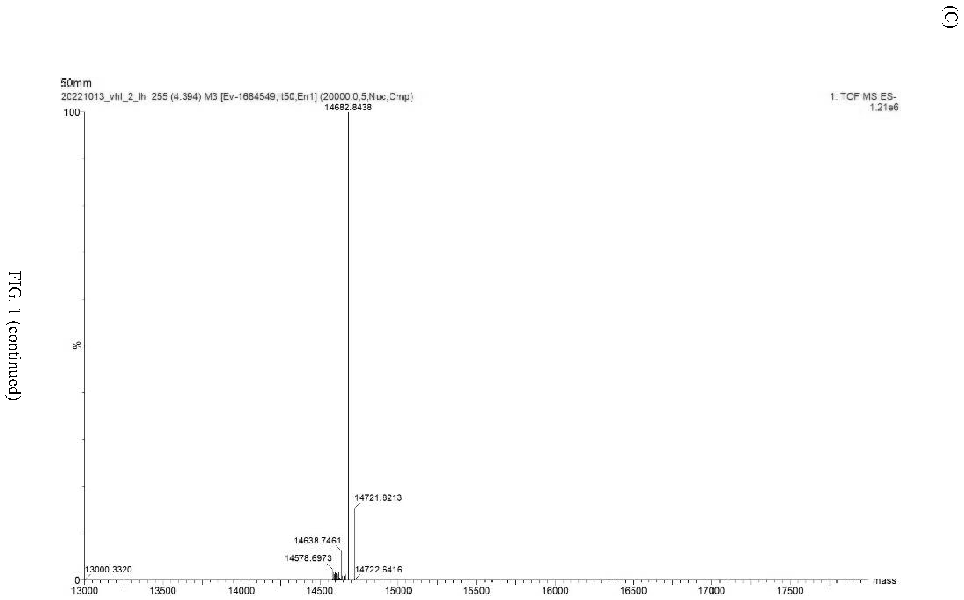

Synthesis of the designed VHL E3 ligand linked to OA-conjugated sclerostin aptamer: The 5'-alkynyl Apc001OA was conjugated to an azide-containing E3 ligand via a copper click reaction, to synthesize the VHL E3 ligand linked to OA-conjugated sclerostin aptamer (FIG. 1A). After completion of the copper click reaction, crude product was purified on a Sephadex^{™} G-25 DNA grade column and HPLC (FIG. 1B), then the purified product was confirmed by ESI-MS (FIG. 1C). The above data show that the VHL E3 ligand linked to sclerostin aptamer had been successfully synthesized.

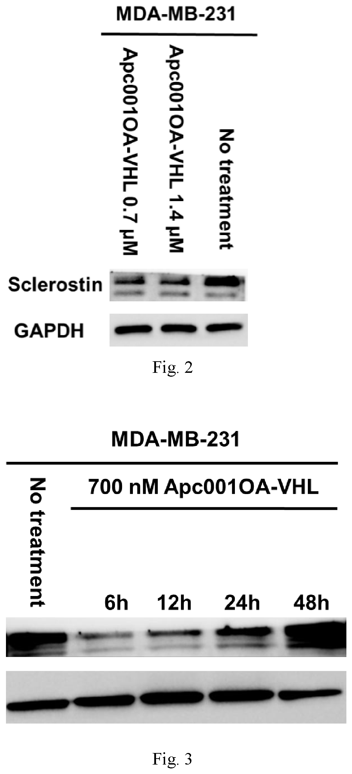

Western blot analysis: Western blot analysis showed that the expression of sclerostin in cells treated with 0.7 and 1.4 µM Apc001OA-VHL was lower than that in PBS-treated group (FIG. 2). It shows that the VHL E3 ligand linked to OA-conjugated sclerostin aptamer can significantly degrade intracellular sclerostin in MDA-MB-231 cells in vitro.

### Example 2. The E3 ligand VHL linked to OA-conjugated sclerostin aptamer showed continuous degradation of intracellular sclerostin in MDA-MB-231 cells in vitro.

The purpose of the present example is to compare differences in expression levels of sclerostin in MDA-MB-231 cells after treatment with 0.7 µM Apc001OA-VHL for 6 h, 12 h, 24 h and 48 h by Western blot analysis.

### Experimental Design

The MDA-MB-231 cells were seeded at a density of 4×10 ⁵ per well in a 6-well plate and incubated overnight. The cells were incubated with 0.7 µM Apc001OA-VHL at 37°C and collected at 6, 12, 24, and 48 hours respectively. Then, the supernatant was removed and protein extract was isolated from cells. Finally, the protein sample was analyzed by Western blotting.

### Assessment Protocol

Western blotting analysis: All protein samples were separated by denaturing 10% SDS-PAGE gel set at 80 V for 30 minutes and then at 120 V for 1 hour. The gel was transferred to a 0.2 µm PVDF membrane (Bio-rad) by electroblotting, set at 350 A for 60 minutes. All membranes were blocked with 5% blotting grade block agent (Bio-Rad) containing 0.1% Tween 20 (TBST) for one hour at room temperature, followed by overnight incubation with primary antibody at 4°C. The membranes were incubated with secondary antibody conjugated with peroxidase at room temperature for 1 hour. According to the manufacturer's instructions, bound antibodies were visualized by enhanced chemiluminescence system (ECL) (Bio-Rad). Sclerostin was detected by anti-sclerostin polyclonal antibody (Abcam, #ab85799). GAPDH was used as a control and detected by anti-GAPDH monoclonal antibody (Santa Cruz, # sc365062). All protein blot images were processed using Image Lab software.

### Results and Discussion

Western blot analysis showed that the level of sclerostin in cells treated with Apc001OA-VHL was significantly lower than that in cells treated with PBS within 24 hours (FIG. 3). Western blot analysis showed the lowest intracellular sclerostin level was observed after 6 hours of treatment with 0.7 µM Apc001OA-VHL. After VHL treatment, the level of sclerostin in cells gradually recovered from 12 h to 48 h (FIG. 3). This may be explained with the following two reasons. One may be related to the re-synthesis of sclerostin in MDA-MB-231 cells. Another possibally is the instability of Apc001OA-VHL in MDA-MB-231 cells. These data indicate that the designed VHL E3 ligand linked to OA-conjugated sclerostin aptamer can degrade sclerostin in MDA-MB-231 cells in vitro for 24 hours.

### Example 3. In Vitro Inhibition of the Proliferation and Migration of MDA-MB-231 Cells by VHL E3 ligand linked to OA-conjugated sclerostin aptamer

Object 1 of the present example: to compare differences in cell proliferation between MDA-MB-231 cells treated with Apc001OA, Apc001OA-VHL and PBS respectively by colony formation in vitro.

Object 2 of the present example is to compare differences in cell migration between MDA-MB-231 cells treated with Apc001OA, Apc001OA-VHL and PBS by an ex vivo Transwell experiment.

### Experimental Design

### Experimental Design of Object 1:

The MDA-MB-231 cells were seeded in a 6-well plate and incubated overnight. The cells were incubated with Apc001OA-VHL (700 nM), Apc001OA (700 nM) and PBS at 37°C respectively. The culture medium was replaced every two days. After 9 days, the colonies were analyzed.

### Experimental Design of Object 2:

The MDA-MB-231 cells were suspended in serum-free DMEM medium, and then the cells were dispersed into upper chambers of 24 transwells. 700 µl of DMEM medium supplemented with 10% FBS was added to each well in the lower 24 well plate. The cells were incubated with Apc001OA-VHL (700 nM), Apc001OA (700 nM) and PBS at 37°C for 48 hours. At the end of incubation, the number of migrated cells in each transwell chamber was analyzed.

### Assessment Protocol

Colony formation assay: MDA-MB-231 cells were trypsinized and counted, then 1000 cells were seeded in each well of a 6-well plate containing complete DMEM medium. Then, the cells were treated with PBS, Apc001OA-VHL and Apc001OA respectively. The medium was replaced every 2 days until visible colonies formed. After 9 days, the cells were fixed with 4% PFA for 10 minutes and stained with crystal violet for 20 minutes (Beyotime). An image was shot by a camera.

Migration Assay: MDA-MB-231 cells were suspended in serum-free DMEM medium and counted, then 5×10 ⁵ cells were distributed into the upper chamber of 24 transwells. 700 µl of DMEM medium supplemented with 10% FBS was added to each well in the lower 24 well plate. The cells were incubated with Apc001OA-VHL, Apc001OA and PBS at 37°C for 48 hours. At the end of incubation, cells were fixed with 4% PFA for 10 min and stained with crystal violet (Beyotime) for 20 min. The number of migrated cells in each transwell chamber was analyzed by microscope.

### Results and Discussion

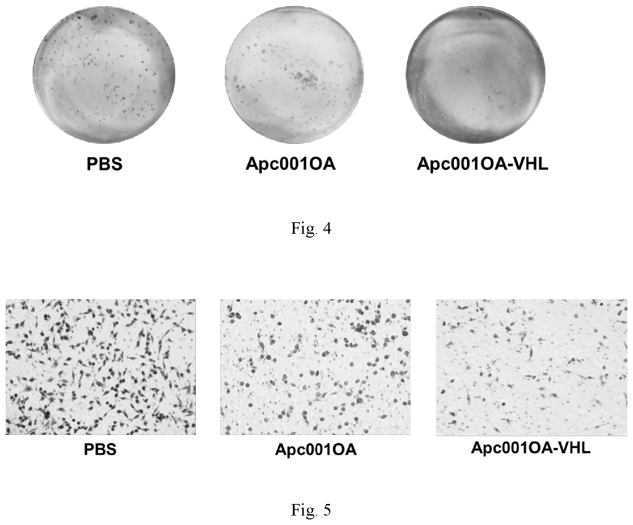

Colony formation assay showed that the number of colonies formed in the group treated with 700 nM Apc001OA-VHL was less than those in PBS and 700 nM Apc001OA-treated groups (FIG. 4). Migration analysis showed that the 700 nM Apc001OA-VHL treatment group displayed fewer migrated cells than the PBS and 700 nM Apc001OA treatment groups (FIG. 5). The above data show that the VHL E3 ligand linked to OA-conjugated sclerostin aptamer (Apc001OA-VHL) can inhibit cell proliferation and migration of MDA-MB-231 cells in vitro.

### Example 4. Inhibition of MDA-MB-231 Cell Viability by VHL E3 ligand linked to OA-conjugated sclerostin aptamer in Vitro

The object of the present example is to compare the difference in cell viability between MDA-MB-231 cells treated with Apc001OA, Apc001OA-VHL and PBS by WST-8 assay.

### Assessment Protocol

Cell viability was assessed by WST-8 assay based on lactate dehydrogenase (Dojindo Molecular Technologies) using Tecan Infinite M1000 multi-mode ELISA reader (Tecan, Morrisville, NC) (Qin et al., 2018). The WST-8 reagent was added to a 96-well culture plate and incubated at 37°C for 1 hour, and read at 450 nm. The readings were normalized to PBS-treated cells.

### Results and Discussion

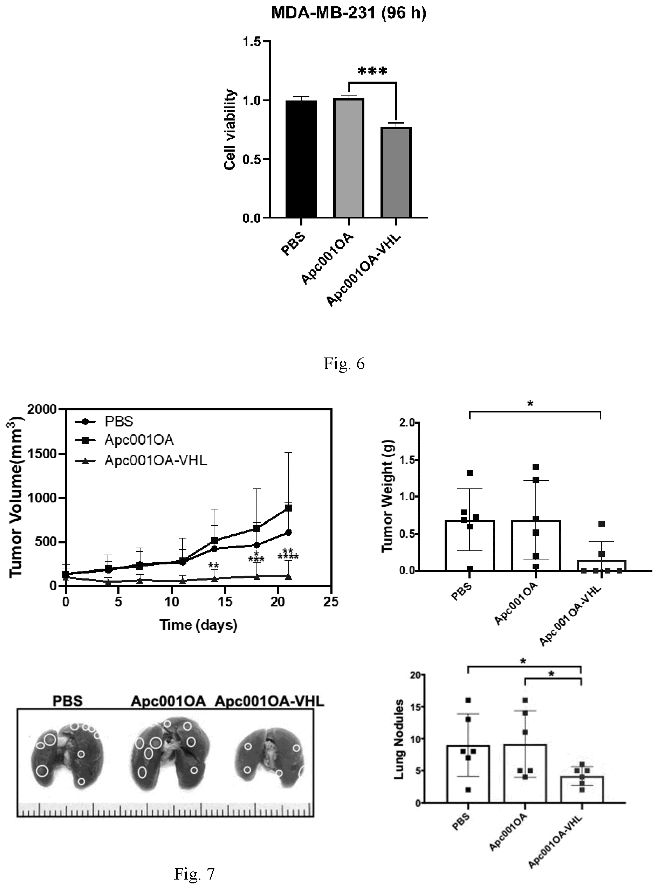

The WST-8 analysis showed that the cell viability of the group treated with 700 nM Apc001OA-VHL was lower than that of PBS and 700 nM Apc001 OA-treated groups (FIG. 6). The above data showed that the designed VHL E3 ligand linked to OA-conjugated sclerostin aptamer (Apc0010A-VHL) could inhibit cell viability of MDA-MB-231 cells in vitro.

### Example 5. VHL E3 ligand linked to OA-conjugated sclerostin aptamer (Apc001OA-VHL) Inhibits Tumor Growth in Subcutaneous Mouse Model Inoculated with MDA-MB-231 Cells and Metastasis of Tumor in In Situ Mouse Model of 4T1 Cells

### Assessment Protocol

Subcutaneous mouse model of MDA-MB-231. For the breast fat pad injection experiment, all 6-8-week-old female athymic nude mice were anesthetized with 2.5% isoflurane and then injected subcutaneously (s.c.) with MDA-MB-231 (2×10 ⁶) using a single-cell suspension in 50 µl of Matrigel: PBS (1:1). The tumor size was monitored by measuring the length (L) and width (W) of the tumor with a caliper, and calculating the volume of the tumor using the formula: V=L×W²/2. A tumor growth curve was constructed to evaluate the progression of primary tumors. When the total tumor volume exceeded 1,000 mm³, all experiments with individual mice were terminated, or if the tumors ulcerated, the experiments were terminated earlier. At the end, the primary tumors were excised, photographed and weighed.

In situ mouse model of 4T1. All eight-week-old female Balb/c mice were anesthetized with 2.5% isoflurane, and then 4T1-luc sost WT cells (1×10 ⁵) were subcutaneously injected (s.c.) into the mammary gland in the abdomen. After all the experiments on individual mice were finished, lung tissues of the mice were taken and counted for lung nodules.

### Results and Discussion

To study the effect of Apc001OA-VHL on in vivo TNBC progression, mice models inoculated with MDA-MB-231 cells were treated with PBS, Apc001OA and Apc001OA-VHL respectively. The treatment regimen of mice inoculated with MDA-MB-231 cells was described. The results of tumor volume measurement (FIG. 7) showed that the tumor volume in Apc001OA-VHL group was significantly smaller than those in PBS (p<0.001) and Apc001OA groups (p<0.01). The results of tumor weight measurement (FIG. 7) showed that the tumor weight in Apc001OA-VHL group was significantly lighter than those in PBS group (p<0.01) and Apc001OA group (p<0.05). The above data showed that Apc001OA-VHL significantly inhibited the tumor progression in a mouse model inoculated with MDA-MB-231 cells.

To study the effect of Apc001OA-VHL on in vivo TNBC metastasis, mice models inoculated with 4T1 cells were treated with PBS, Apc001OA and Apc001OA-VHL respectively. The visible nodules in lungs were counted to quantify lung metastases. The statistical results showed that the number of lung metastatic nodules in Apc001OA-VHL group was significantly lower than those in PBS and Apc001OAgroups (FIG. 7). This indicates that Apc001OA-VHL significantly inhibits the degradation of sclerostin in cells, thereby suppressing tumor metastasis in an in situ mouse model inoculated with 4T1 cells.

### Example 6: Synthesis and Application of Nucleic Acid-Based Molecule (LYTAC) for Targeted Extracellular and Systemic Protein Degradation

At present, targeted protein degradation (TPD) has become a promising targeted therapy technology, which is mainly based on two protein degradation pathways: proteasome pathway and lysosome pathway. For the proteasome pathway, the bifunctional molecule that links a target protein to E3 enzyme is called as proteolysis targeting chimera (PROTAC), which degrades the target protein within the cell by using the ubiquitin-proteasome system through a series of enzymes. For the lysosomal pathway, a well-known bifunctional molecule that links a target protein to a transmembrane receptor is LYTAC (lysosome-targeting chimera), which mediates endocytosis of the target protein into lysosome after endocytosis. Then, the lysosomal microenvironment degrades the target transmembrane or extracellular protein, which is a good complement of PROTAC.

Inspired by the earliest PROTAC molecule, other TPD technologies such as autophagy-targeting chimera (AUTAC), specificity and non-genetic apoptosis inhibitor protein-dependent proteolysis-targeting chimera (SNIPER) and autophagosome-binding compound (ATTEC) are emerging and constantly developing. The trivalent N-acetylgalactosamine antibody conjugate (GalNAc-Antibody) LYTAC developed by the Bertozzi group targets and degrades proteins through binding to asialoglycoprotein receptor (ASGPR), which has drawn widespread attention in the academic community. ASGPR is highly expressed on the surface of hepatocytes, can recognize GalNAc moiety and internalize its conjugates into cellular lysosomes via clathrin-mediated endocytosis, making it a good liver-specific lysosomal targeting receptor. It has been widely used for liver-targeting oligonucleotide delivery by GalNAc recognition. In the work of Bertozzi's group, GalNAc-antibody LYTAC successfully degrades epidermal growth factor receptor (EGFR) in vitro, and GalNAc-pipetide LYTAC successfully degrades integrins in vitro. However, the preparation of chimeras by conjugating antibodies with GalNAc molecules is not only complex and time-consuming but also uncertain in the number and position of conjugation. In addition, the large molecular weight (usually about 150 kD) results in poor cell internalization.

Nucleic acid aptamer is a short fragment of DNA or RNA, which can recognize and bind to the target molecule (usually protein) by specific 3D structure. These fragments are selected from random synthesized oligodeoxyribonucleotide or oligonucleotide library using SELEX (Systematic Evolution of Ligands by EXponential enrichment). Compared with small molecular ligands, aptamers often have better selectivity and higher affinity. Compared with antibodies, aptamers have simpler evolution processes, are easier to be modified and adjusted in affinity, have lower immunogenicity, and can be designed and engineered universally. Therefore, aptamers become ideal recognition ligands for their targets. Therefore, more and more aptamers have been developed for use as therapeutic agents and probes.

After the Bertozzi group developed GalNAc-antibody LYTAC, Zhu Zhi's group further developed GalNAc- aptamer to specifically degrade membrane protein tyrosine kinase 7 (PTK7) and extracellular protein platelet-derived growth factor (PDGF) by conjugating GalNAc molecules with corresponding aptamers in hepatocytes. However, they only discussed the feasibility of TPD with GalNAc-Aptamer in vitro. There is an urgent need for specific in vivo application scenarios to validate the drugability of pathogenic protein degradation mediated by GalNAc-Aptamer.

### Assessment Protocol

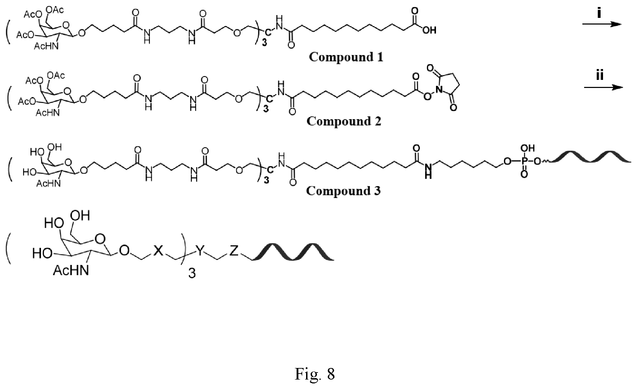

Construction of GalNAc-Apc001 conjugate (FIG. 8): N-hydroxysuccinimide (NHS) (50.7 mg, 0.45 mmol) and 1-ethyl-3-(3-dimethylaminopropyl)carbodiimide (EDCI) (85.7 mg, 0.45 mmol) were added to a solution of tris-GalNAc carboxylic acid derivative (compound 1, WuXi AppTec) (500 mg, 0.3 mmol) in anhydrous dichloromethane (DCM) (20 mL), under nitrogen atmosphere. The mixture was left to stand overnight and then quenched by the addition of saturated NaHCO₃. The residue was rapidly partitioned between DCM (100 mL) and saturated NaHCO₃ (50 mL). The organic layer was washed with saturated NaCl, dried (Na₂SO₄) and evaporated to dryness to give crude product (compound 2) (540 mg, 0.246 mmol, yield: ~82%). Compound 2 was added to the solution of amino-DNA (100 nmol) in triethylamine acetate (TEAA) (1.0 M, 5 mL, pH=8.0), and incubated overnight without further operation. Then, the mixture was added to an ammonium hydroxide solution (10 mL) and heated to 60°C for 3 hours. The solvent was removed under vacuum and the solid residue was redissolved in dH₂O (1 mL). Then, the mixture was purified by high performance liquid chromatography (Agilent 1260). Phase A was acetonitrile (CAN), and phase B was TEAA (50 mM). The Xbride^{®} oligonucleotide BEH C18 OBTM preparation column (2.5 µm, 10 mm×50 mm) was run from 5% to 60% gradient of phase A in 30 minutes at a flow rate of 1.2 mL.min⁻¹, ambient temperature. The purified GalNAc-Apc001 (or GalNAc-scApc001, negative control) was desalted using a Sephadex G25 column to obtain compound 3. The sample was further lyophilized and stored.

Confocal laser scanning microscope (CLSM) imaging: HepG2 and A373 cells were seeded at a density of 8×10 ⁴ on confocal plates in 2 mL of DMEM medium, and cultured for 24 h at 37°C, under humidified air and 5% CO₂. Then, the GalNAc-Apc001-FAM, Apc001-FAM, GalNAc-Scramble, GalNAc-Apc001/Sclerostin-FAM mixture, Apc001/Sclerostin-FAM mixture or GalNAc-Scramble/Sclerostin-FAM mixture of predetermined concentration was added to the medium and cells were incubated for a predetermined time. Then, the cell nucleus was stained with a 1× Hoechst 33342 staining solution (Biyotime), and lysosomes were stained with 50 nM Lysotracker Red (Biyotime) at 37°C for 30 min simultaneously. The cells were gently washed three times with 1× PBS buffer and kept in the 1× PBS buffer throughout the imaging process. Fluorescence imaging of cells was performed using a confocal laser scanning microscope (LEICA TCS SP8) in 60× water immersion mode through selected channels (blue channel: 405 nm; green channel: 488 nm; red channel: 640 nm). Note: for time-dependent imaging, cell nuclei and lysosomes were not stained to obtain better cell viability. At each imaging time point, the culture medium containing fluorescent sample was replaced with normal culture medium and then reversed to further incubate after imaging.

Accumulation and degradation of sclerostin in ASGPR⁺ cells were analyzed by western blot: HepG2 cells were seeded at a density of 1×10 ⁵/well on the confocal plate in 24-well plates, and then cultured for 24 hours under humidified air with 37°C and 5% CO₂. Then, the cells were incubated with a 500 nM GalNAc-Apc001/Sclerostin (1:1) mixture for different times (0 h, 1 h, 2 h, 4 h and 8 h). At 8 h, the culture medium was replaced with normal culture medium to observe the degradation of sclerostin until 18 h. The cells were washed three times with 1× PBS buffer and lysed on ice for 30 minutes in RIPA buffer. The supernatant was collected and the protein concentration was roughly calculated by Nanodrop 2000. Equal amounts of cell lysates were subjected to electrophoresis on 12% SDS-PAGE gels, followed by PVDF membrane transfer and non-specific blocking with 5% skim milk in TBST buffer. The membrane was incubated with primary antibody (diluted 1:1,000 in 5% fat-free milk) overnight at 4°CThe membrane was gently washed three times with 1× TBST buffer on a shaker, and then incubated with secondary antibody (diluted to 1:5000 in 5% skim milk) at room temperature for 1 h. After washing with TBST buffer three times, the membrane was incubated with BeyoECL Plus substrate for 2 min and then imaged by ChemiDoc Imaging System (Bio-Rad).

Assessment of the effect of GalNAc-Apc001 on sclerostin-induced Wnt signaling inhibition: HEK 293 cells were cultured at 37°C in humidified air and 5% CO₂ in Dulbecco's Modified Eagle Medium (DMEM, Omacgene) containing 10% fetal bovine serum (FBS, CellMax) and 1% penicillin-streptomycin (Gibco by Life Technology). The cells were inoculated in a 24-well plate with 0.5 mL of DMEM medium at the density of 1×10 ⁵ cells/well, and cultured for 24 h. The Topflash plasmid (Firefly luciferase, 100 ng/well), the SV40 plasmid (Renilla luciferase, 10 ng/well) and Wnt-1 plasmid (200 ng/well) were co-transfected into cells using Lipofectamine 3000 (1 µL/well, P3000 reagent 1 µL/well, Thermoscientific). In this process, the plasmid and lipofectamine 3000 were mixed in 25 µL of OptiMEM medium (Omacgene) as buffer A. The P3000 reagent was dissolved in 25 µL of OptiMEM medium to serve as buffer B. The buffer A and the buffer B were mixed and incubated for 15 minutes. Then, the mixture was directly added to a culture medium and cultured for 6 hours. Subsequently, the transfection medium was removed and 0.5 ml of fresh DMEM medium containing 100 nM sclerostin, pre-prepared other cells (HepG2, A375 or none), and a sample to be verified (GalNAc-Apc001, Apc001, GalNAc-Scramble or Sclerostin Antibody) at 500 nM were added. After incubating at 37°C for 12 hours, the medium was removed and the culture vessel was shaken at room temperature for 15 minutes to lyse cells in 200 µL of 1× passive lysis buffer (PLB, Promega). Then, 15 µL of the lysate was transferred to a 96-well OptiPlate and luminescence activity was measured by measuring chemiluminescence using an MD SpectraMax i3X multi-mode microplate reader system according to the instructions for the Dual-Luciferase Reporter Assay System (Promega).

Micro-CT analysis: Bone volume and trabecular microstructure analyses were performed on the cortical microstructure of the shaft of left femur, trabeculae of the proximal epiphysis of left tibia and trabeculae of the distal epiphysis of left femur using a Micro-CT (version 6.5, viva CT40, SCANCO Medical AG, Bassersdorf, Switzerland). The femur and tibia images were reconstructed and calibrated with isotropic voxel sizes of 12.5 and 17.5 µm, respectively (70 kVp, 114 µA, 200 ms integration time, 260 threshold, 1200 mg HA/cm³). The same filtering and partitioning values will be used for each measurement. Using the Scanco evaluation software, regions of interest (ROI) were defined for trabecular parameters. For the trabeculae, a central region equivalent to 70% of vertebral body height will be selected and extend from the growth plate end toward the vertebral body from proximal to distal. For proximal tibia and distal femora of mice, 100 consecutive slices starting at the point closest to the growth plate were selected for analysis where two condyles are no longer visible. Small trabecular ROI were manually drawn on 100 consecutive slices to ensure that they are located within the endosteal capsule. Trabecular bone parameters were calculated, including trabecular volume BMD (Tb.vBMD), number of trabeculae (Tb.N) and connectivity density of trabeculae (Tb.conn.D); For the femoral midshaft, 100 consecutive slices were measured at the precise center and distal 50% of the femoral length using an automatic threshold algorithm. The trabeculaes in contact with the cortical bone were manually removed from the ROI. The parameters of the cortical bone were calculated, including Ct. periosteal circumference, Ct. endocortical circumference and Ct. bone strength index.

Statistical analysis: all variables were expressed as mean ± standard deviation. One-way ANOVA was performed using Tukey post-hoc test to determine inter-group differences in the study variables, including ex vivo Wnt-induced signaling, ex vivo bone formation biomarker mRNA levels, micro-CT parameters, histomorphometric parameters and mechanical testing. All statistical data were analyzed using Origin 2019b and GraphPad Prism, P<0.05 was statistically significant. In in vivo experiments, the researchers randomly divided the animals into groups. Animals with poor physical conditions were excluded.

### Results and Discussion

### Transmembrane Transport Potential of GalNAc-Apc001 Conjugate in ASGPR⁺ Cells

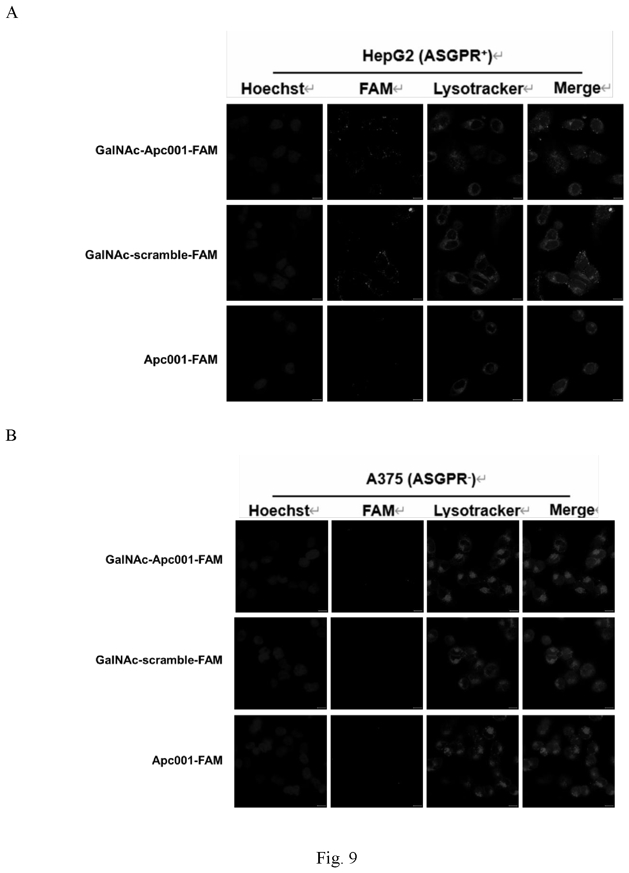

Tri-GalNAc has been widely used to deliver a variety of cargoes to hepatocytes via the asialoglycoprotein receptor (ASGPR)-mediated cell uptake. To determine the transmembrane transport potential of GalNAc-Apc001 conjugate, GalNAc-Apc001 was fluorescently labeled with a FAM group at 3'-end, i.e., GalNAc-Apc001-FAM. Apc001-FAM and the scrambled sequence GalNAc-Scramble-FAM were used as controls. The HepG2 cell is a hepatoma cell line, which reportedly has high level of ASGPR expression on the surface of the cells and is used as positive cells. In contrast, human melanoma cell line A375 cells with the lowest expression of ASGPR were used as negative cells. Confocal laser scanning microscopy imaging was performed to determine the transmembrane transport potential of GalNAc-Apc001 conjugate in ASGPR⁺ cells. After incubating HepG2 cells with GaINAc-Apc001-FAM for 2 hours, the fluorescence signal was significantly increased. However, no increase in fluorescence signal was observed in A375 cells (FIG. 9), indicating that internalization of GalNAc-Apc001 is cell-specific. Both GalNAc-Apc001-FAM and GalNAc-Scramble-FAM treatment increased the fluorescent signal in HepG2 cells, but no increase of the fluorescent signals was observed in Apc001-FAM-treated group, indicating that internalization is GalNAc-dependent (FIG. 9). In addition, the internalized fluorescent signal in GalNAc-treated HepG2 cells co-localizes with Lysotracker Red signals for visualizing acidic lysosomes, indicating that GalNAc directs its cargo to an acidic compartment of ASGPR⁺ cells. In conclusion, GalNAc can mediate cell-specific internalization and transport FAM-labeled Apc001 into lysosomes of ASGPR⁺ cells.

### Targeted Degradation of sclerostin by GaINAc-Apc001 Conjugate in ASGPR⁺ Cells

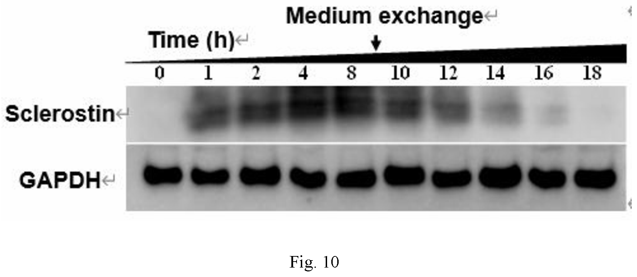

Western blotting for sclerostin (with GAPDH as an internal control) was performed to monitor the change of content in HepG2 cells. As shown in FIG. 10, the time-dependent accumulation of sclerostin in HepG2 cells was observed under sample medium (for 1 hour to 8 hours). After the sample medium was replaced with a normal medium at 8 hours, it was observed that sclerostin gradually degraded (8 h to 18 h). It demonstrates that GalNAc-Apc001 can mediate the cell-specific degradation of sclerostin in ASGPR⁺ cells.
In the presence of ASGPR⁺ cells in vitro, GaINAc-Apc001 can greatly reduce the inhibitory effect of sclerostin on Wnt signaling.

The HEK 293 cell line which has been widely used as a cell tool line for Wnt signaling research, was incubated with Top Flash plasmid (Firefly luciferase, TCF/LEF response element), SV40 plasmid (Renilla luciferase, internal control) and Wnt-1 plasmid for 6 hours. After the medium was replaced, sclerostin was directly added to the medium to construct a model in which Wnt signaling induced by Wnt-1 was greatly inhibited. After the cells are treated with a sclerostin inhibitor, Wnt signaling will be reactivated. As expected, the data show that Wnt-1 induced luciferase signaling is greatly inhibited by sclerostin, confirming the inhibitory effect of sclerostin on Wnt signaling. To assess the effect of GalNAc-Apc001 on Wnt signaling inhibition induced by sclerostin, pre-prepared other cells (HepG2, A375 or none) and sample mixtures to be verified (GalNAc-Apc001, Apc001, GalNAc-Scramble, or sclerostin antibody) were added into the medium after transfection. After incubation for 12 hours, the cells were lysed and luciferase activity was measured by a dual-reporter assay. Surprisingly, the relative luciferase activity of GalNAc-Apc001-treated group was restored to 96% of that induced by Wnt-1 in the presence of HepG2 cells, which is nearly 15% higher than that treated with sclerostin antibody Romosozumab and 39% higher than that treated with Apc001. However, no significant difference was observed between GalNAc-Apc001 and Apc001 in the presence of A375 cells or without additional cells. This indicates that GalNAc-Apc001 mediates the reactivation of Wnt signals in ASPGPR+ cells specifically. In addition, no Wnt signal reactivation was observed in the GalNAc-scramble treatment group regardless of whether HepG cells or A375 cells were present or not, indicating that Wnt signal reactivation is dependent on Apc001.

### GalNAc conjugation facilitates the modified aptamer to promote bone anabolism in Osteogenesis Imperfecta mice (Col1a2^{+/}^{-G610C})

To evaluate the effect of GalNAc-modified aptamer on promoting bone anabolism in OI mice, bone trabeculae at distal metaphysis of femur were analyzed by micro-computed tomography (micro-CT). Subcutaneous injections of PBS (OI-Veh), GalNAc-Apc001 25 mg/kg, and sclerostin antibody 25 mg/kg were performed in OI mice at the age of six to eight weeks (once a week for 12 weeks). Wild-type mice were injected subcutaneously with PBS (WT-Vehicle) once per week for 12 weeks. The *Col1a2*^{*+*/*-*}/G610C mice were sacrificed before OI-baseline (OI-BS) treatment.

### References:

1. Cao, C., He, M., Wang, L., He, Y., and Rao, Y. (2022). Chemistries of bifunctional PROTAC degraders. Chem Soc Rev. 51(16), 7066-7114. doi: 10.1039/d2cs00220e.
2. Garber, K. (2022). The PROTAC gold rush. Nat. Biotechnol. 40(1), 12-16. doi: 10.1038/s41587-021-01173-2.
3. Hesse, E., Schroder, S., Brandt, D., Pamperin, J., Saito, H., and Taipaleenmaki, H. (2019). Sclerostin inhibition alleviates breast cancer-induced bone metastases and muscle weakness. JCI Insight 5(9). doi: 10.1172/jci.insight.125543.
4. Patil, K.M., Chin, D., Seah, H.L., Shi, Q., Lim, K.W., and Phan, A.T. (2021). G4-PROTAC: targeted degradation of a G-quadruplex binding protein. Chem Commun (Camb) 57(95), 12816-12819. doi: 10.1039/d1cc05025g.
5. Sakamoto, K.M., Kim, K.B., Kumagai, A., Mercurio, F., Crews, C.M., and Deshaies, R.J. (2001). Protacs: chimeric molecules that target proteins to the Skp1-Cullin-F box complex for ubiquitination and degradation. Proc. Natl. Acad. Sci. U. S. A. 98(15), 8554-8559. doi: 10.1073/pnas.141230798.
6. Wang, L., Yu, Y., Ni, S., Li, D., Liu, J., Xie, D., et al. (2022). Therapeutic aptamer targeting sclerostin loop3 for promoting bone formation without increasing cardiovascular risk in osteogenesis imperfecta mice. Theranostics 12(13), 5645-5674. doi: 10.7150/thno.63177.
7. Yu, Y., Wang, L., Ni, S., Li, D., Liu, J., Chu, H.Y., et al. (2022). Targeting loop3 of sclerostin preserves its cardiovascular protective action and promotes bone formation. Nat Commun 13(1), 4241. doi: 10.1038/s41467-022-31997-8.
8. Zhang, G., Guo, B., Wu, H., Tang, T., Zhang, B.T., Zheng, L., et al. (2012). A delivery system targeting bone formation surfaces to facilitate RNAi-based anabolic therapy. Nat Med 18(2), 307-314. doi: 10.1038/nm.2617.
9. Zhang, Y., Zhang, H., Chan, D.W.H., Ma, Y., Lu, A., Yu, S., et al. (2022). Strategies for developing long-lasting therapeutic nucleic acid aptamer targeting circulating protein: The present and the future. Front Cell Dev Biol 10, 1048148. doi: 10.3389/fcell.2022.1048148.
10. Zhang, G., Guo, B., Wu, H., Tang, T., Zhang, B.T., Zheng, L., et al. (2012). A delivery system targeting bone formation surfaces to facilitate RNAi-based anabolic therapy. Nat Med 18(2), 307-314. doi: 10.1038/nm.2617.
11. Peng, L., Zhang, Z., Lei, C., Li, S., Zhang, Z., Ren, X., et al. (2019). Identification of New Small-Molecule Inducers of Estrogen-related Receptor alpha (ERRalpha) Degradation. ACS Med Chem Lett 10(5), 767-772. doi: 10.1021/acsmedchemlett.9b00025.
12. Winter, G.E., Buckley, D.L., Paulk, J., Roberts, J.M., Souza, A., Dhe-Paganon, S., et al. (2015). Phthalimide conjugation as a strategy for in vivo target protein degradation. Science 348(6241), 1376-1381. doi: 10.1126/science.aab1433.
13. Liu, J., Chen, H., Kaniskan, H.U., Xie, L., Chen, X., Jin, J., et al. (2021a). TF-PROTACs Enable Targeted Degradation of Transcription Factors. Journal of the American Chemical Society 143(23), 8902-8910. doi: 10.1021/jacs.1c03852.
14. Zhang, L., Li, L., Wang, X., Liu, H., Zhang, Y., Xie, T., et al. (2022a). Development of a novel PROTAC using the nucleic acid aptamer as a targeting ligand for tumor selective degradation of nucleolin. Mol Ther Nucleic Acids 30, 66-79. doi: 10.1016/j.omtn.2022.09.008.
15. Qin, C., Hu, Y., Zhou, B., Fernandez-Salas, E., Yang, C.Y., Liu, L., et al. (2018). Discovery of QCA570 as an Exceptionally Potent and Efficacious Proteolysis Targeting Chimera (PROTAC) Degrader of the Bromodomain and Extra-Terminal (BET) Proteins Capable of Inducing Complete and Durable Tumor Regression. J. Med. Chem. 61(15), 6685-6704. doi: 10.1021/acs.jmedchem.8b00506.
16. Zhang, G.; Guo, B.; Wu, H.; Tang, T.; Zhang, B. T.; Zheng, L.; He, Y.; Yang, Z.; Pan, X.; Chow, H.; To, K.; Li, Y.; Li, D.; Wang, X.; Wang, Y.; Lee, K.; Hou, Z.; Dong, N.; Li, G.; Leung, K.; Hung, L.; He, F.; Zhang, L.; Qin, L., A delivery system targeting bone formation surfaces to facilitate RNAi-based anabolic therapy. Nat Med 2012, 18 (2), 307-14.
17. Peng, L.; Zhang, Z.; Lei, C.; Li, S.; Zhang, Z.; Ren, X.; Chang, Y.; Zhang, Y.; Xu, Y.; Ding, K., Identification of New Small-Molecule Inducers of Estrogen-related Receptor alpha (ERRalpha) Degradation. ACS Med Chem Lett 2019, 10 (5), 767-772.
18. Winter, G. E.; Buckley, D. L.; Paulk, J.; Roberts, J. M.; Souza, A.; Dhe-Paganon, S.; Bradner, J. E., Phthalimide conjugation as a strategy for in vivo target protein degradation. Science 2015, 348 (6241), 1376-1381.
19. Liu, J.; Chen, H.; Kaniskan, H. U.; Xie, L.; Chen, X.; Jin, J.; Wei, W., TF-PROTACs Enable Targeted Degradation of Transcription Factors. Journal of the American Chemical Society 2021, 143 (23), 8902-8910.
20. Zhang, L.; Li, L.; Wang, X.; Liu, H.; Zhang, Y.; Xie, T.; Zhang, H.; Li, X.; Peng, T.; Sun, X.; Dai, J.; Liu, J.; Wu, W.; Ye, M.; Tan, W., Development of a novel PROTAC using the nucleic acid aptamer as a targeting ligand for tumor selective degradation of nucleolin. Mol Ther Nucleic Acids 2022, 30, 66-79.
21. Adhikary, S.; Chakravarti, D.; Terranova, C.; Sengupta, I.; Maitituoheti, M.; Dasgupta, A.; Srivastava, D. K.; Ma, J.; Raman, A. T.; Tarco, E.; Sahin, A. A.; Bassett, R.; Yang, F.; Tapia, C.; Roy, S.; Rai, K.; Das, C., Atypical plant homeodomain of UBR7 functions as an H2BK120Ub ligase and breast tumor suppressor. Nat Commun 2019, 10 (1), 1398.
22. Si, W.; Zhou, J.; Zhao, Y.; Zheng, J.; Cui, L., SET7/9 promotes multiple malignant processes in breast cancer development via RUNX2 activation and is negatively regulated by TRIM21. Cell Death Dis 2020, 11 (2), 151.
23. Zhao, L.; Zhao, J.; Zhong, K.; Tong, A.; Jia, D., Targeted protein degradation: mechanisms, strategies and application. Signal Transduction and Targeted Therapy 2022, 7 (1), 113.
24. Békés, M.; Langley, D. R.; Crews, C. M., PROTAC targeted protein degraders: the past is prologue. Nature Reviews Drug Discovery 2022, 21 (3), 181-200.
25. Garber, K., The lysosomal degraders. Nature Biotechnology 2022, 40 (12), 1709-1713.
26. Takahashi, D.; Moriyama, J.; Nakamura, T.; Miki, E.; Takahashi, E.; Sato, A.; Akaike, T.; Itto-Nakama, K.; Arimoto, H., AUTACs: Cargo-Specific Degraders Using Selective Autophagy. Molecular Cell 2019, 76 (5), 797-810.e10.
27. Naito, M.; Ohoka, N.; Shibata, N., SNIPERs-Hijacking IAP activity to induce protein degradation. Drug Discovery Today: Technologies 2019, 31, 35-42.
28. Li, Z.; Zhu, C.; Ding, Y.; Fei, Y.; Lu, B., ATTEC: a potential new approach to target proteinopathies. Autophagy 2020, 16 (1), 185-187.
29. Ahn, G.; Banik, S. M.; Miller, C. L.; Riley, N. M.; Cochran, J. R.; Bertozzi, C. R., LYTACs that engage the asialoglycoprotein receptor for targeted protein degradation. Nature Chemical Biology 2021, 17 (9), 937-946.
30. D'Souza, A. A.; Devarajan, P. V., Asialoglycoprotein receptor mediated hepatocyte targeting - Strategies and applications. Journal of Controlled Release 2015, 203, 126-139.
31. Cui, H.; Zhu, X.; Li, S.; Wang, P.; Fang, J., Liver-Targeted Delivery of Oligonucleotides with N-Acetylgalactosamine Conjugation. ACS Omega 2021, 6 (25), 16259-16265.
32. Debacker, A. J.; Voutila, J.; Catley, M.; Blakey, D.; Habib, N., Delivery of Oligonucleotides to the Liver with GalNAc: From Research to Registered Therapeutic Drug. Molecular Therapy 2020, 28 (8), 1759-1771.
33. Springer, A. D.; Dowdy, S. F., GalNAc-siRNA Conjugates: Leading the Way for Delivery of RNAi Therapeutics. Nucleic Acid Therapeutics 2018, 28 (3), 109-118.
34. Zhou, Y.; Teng, P.; Montgomery, N. T.; Li, X.; Tang, W., Development of Triantennary N-Acetylgalactosamine Conjugates as Degraders for Extracellular Proteins. ACS Central Science 2021, 7 (3), 499-506.
35. Shraim, A. S.; Abdel Majeed, B. A.; Al-Binni, M. A.; Hunaiti, A., Therapeutic Potential of Aptamer-Protein Interactions. ACS Pharmacol Transl Sci 2022, 5 (12), 1211-1227.
36. Zhou, J.; Rossi, J., Aptamers as targeted therapeutics: current potential and challenges. Nature Reviews Drug Discovery 2017, 16 (3), 181-202.
37. Dunn, M. R.; Jimenez, R. M.; Chaput, J. C., Analysis of aptamer discovery and technology. Nature Reviews Chemistry 2017, 1 (10), 0076.
38. Röthlisberger, P.; Hollenstein, M., Aptamer chemistry. Advanced Drug Delivery Reviews 2018, 134, 3-21.
39. Wu, L.; Wang, Y.; Xu, X.; Liu, Y.; Lin, B.; Zhang, M.; Zhang, J.; Wan, S.; Yang, C.; Tan, W., Aptamer-Based Detection of Circulating Targets for Precision Medicine. Chemical Reviews 2021, 121 (19), 12035-12105.
40. Wu, Y.; Lin, B.; Lu, Y.; Li, L.; Deng, K.; Zhang, S.; Zhang, H.; Yang, C.; Zhu, Z., Aptamer-LYTACs for Targeted Degradation of Extracellular and Membrane Proteins. Angewandte Chemie International Edition 2023, 62 (15), e202218106.

### SEQUENCE INFORMATION

| SEQ ID NO | Name | Sequence | K_{d} (nM) |
|---|---|---|---|
| 1 | aptsc132 | CCCAGACGAGACACCTCATGCTTTTCCCCGGGGGAGGGGTAT | 42.2 |
| 2 | aptsc16 | CGTACGGTCGACGCTAGCTGGAAGGGTGGGGGCGGGGGGTC CTCGCCTCGAACGTACGCACGTGGAGCTCGGATCC | 4.2 |
| 3 | aptscl9 | CGTACGGTCGACGCTAGCTGGGGGTAGGGGGACCCTGGCTA GTTAGTCACCGTTTCGACACGTGGAGCTCGGATCC | 3.4 |
| 4 | aptscl15 | CGTACGGTCGACGCTAGCGAGGGGGCCAACTATGCTTAGTG GGGGGGTTGACCGTATCCACGTGGAGCTCGGATCC | 4 |
| 5 | aptscl46 | CGTACGGTCGACGCTAGCGGGTGGATTAAGGGGGCCCCGTC TGTAGGCGATTGGCGAACACGTGGAGCTCGGATCC | 45.6 |
| 6 | aptscl51 | CGTACGGTCGACGCTAGCTGGGGGTAGGGGGTCCTTGGCTA CGGTGATTCGGATGTGACACGTGGAGCTCGGATCC | 62.2 |
| 7 | aptscl1 | CGTACGGTCGACGCTAGCTCTTGTTCATCGATCCTACGCACG TGGAGCTCGGATCC | 0.02 |
| 8 | aptscl2 | CGTACGGTCGACGCTAGCCAGCCAAAGACAGAGATGCACGT GGAGCTCGGATCC | 0.006 |
| 9 | aptscl3 | CGTACGGTCGACGCTAGCTGTTGTACATCGCCTTACGCACGT GGAGCTCGGATCC | 0.04 |
| 10 | aptscl5 | CGTACGGTCGACGCTAGCTGTTGTTCATCGACTTGACGCACG TGGAGCTCGGATCC | 0.005 |
| 11 | aptscl8 | CGTACGGTCGACGCTAGCCGGCGAAATTGCTACCACGTGGA GCTCGGATCC | 0.005 |
| 12 | aptscl12 | CGTACGGTCGACGCTAGCCGTTGACTCGTTGCTACACGTGGA GCTCGGATCC | 0.005 |
| 13 | aptsci16 | CGTACGGTCGACGCTAGCTGGTTTCATCGATCGTATCCACGT GGAGCTCGGATCC | 0.61 |
| 14 | aptscl22 | CGTACGGTCGACGCTAGCTGGGTTTATCGACTAGTTCCACGT GGAGCTCGGATCC | 0.76 |
| 15 | aptscl29 | CGTACGGTCGACGCTAGCCGGAGACCTGAGGTTCACGTGGA GCTCGGATCC | 0.28 |
| 16 | aptscl32 | CGTACGGTCGACGCTAGCTGTGTTAATCGCCGTACCTCCACG TGGAGCTCGGATCC | 0.18 |
| 17 | aptscl56 | CGGGGTGTGGGTTCGTCGTTAGCTTGATTTGGCAGCTGCC | 45 |

## Claims

1. An aptamer conjugate comprising i) an aptamer that specifically binds to sclerostin, and ii) a specific ligand of E3 ubiquitin ligase (E3) and/or a specific ligand of asialoglycoprotein receptor (ASGPR).

2. The aptamer conjugate of Claim 1, wherein the aptamer that specifically binds to sclerostin comprises
(i) a nucleotide sequence having at least about 90%, at least about 91%, at least about 92%, at least about 93%, at least about 94%, at least about 95%, at least about 96%, at least about 97%, at least about 98%, or at least about 99% identity to any of SEQ ID NOs: 1-17; or
ii) at least 30, at least 35, at least 40, at least 45, at least 50 or more consecutive nucleotides in any one of SEQ ID NOs: 1 to 17; or
iii) any nucleotide sequence in SEQ ID NOs: 1 to 17, preferably the nucleotide sequence of SEQ ID NO: 17.

3. The aptamer conjugate of Claim 1 or 2, wherein the aptamer that specifically binds to sclerostin has a Kd (dissociation constant) of less than 100 nM, preferably less than 50 nM, more preferably less than 40 nM, more preferably less than 30 nM, more preferably less than 20 nM, more preferably less than 10 nM, or less.

4. The aptamer conjugate of any one of claims 1 to 3, wherein the aptamer that specifically binds to sclerostin is a modified aptamer that can comprise one or more modifications conferring enhanced nuclease resistance to said aptamer and/or extending the in vivo half-life of said aptamer.

5. The aptamer conjugate of claim 4, wherein the modification comprises a 3' inverted deoxythymidine (3' idT) modification.

6. The aptamer conjugate of claim 4, wherein the modification comprises replacing one or more naturally occurring nucleotides with a modified nucleotide selected from a 2'-fluoro-, 2'-methoxyethyl-, 2'-methoxy- and/or 2'-allyloxy-modified nucleotide, preferably a 2'-methoxy-modified nucleotide.

7. The aptamer conjugate of claim 4, wherein the modification comprises an inter-nucleotide modification, for example, an inter-nucleotide phosphorothioate bond modification.

8. The aptamer conjugate of claim 4, wherein the aptamer comprises a 2'-methoxy (2'-OMe) modification and/or a 3' inverted deoxythymidine (3'idT) modification.

9. The aptamer conjugate of any one of claims 1 to 8, wherein the aptamer is further conjugated with a fatty acid.

10. The aptamer conjugate of claim 9, wherein the fatty acid is selected from palmitic acid (PA), dodecanedioic acid (DA), tetradecanedioic acid, hexadecanedioic acid, stearic acid (SA), octadecanedioic acid, lauric acid, eicosapentaenoic acid (EPA), docosahexaenoic acid (DHA), and arachidonic acid (ARA), preferably, the fatty acid is octadecanedioic acid.

11. The aptamer conjugate of claim 1 to 10, wherein the specific ligand of E3 is selected from von Hippel-Landau (VHL), murine double minute 2 (MDM2) and cereblon (CRBN); preferably, the specific ligand of E3 is VHL, for example, the VHL comprises a structure shown in the following formula:

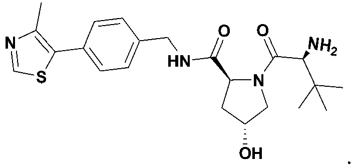

12. The aptamer conjugate of any one of claims 1 to 11, wherein the specific ligand of E3 is conjugated with the aptamer via a linker, for example, conjugated to the 5' end of the aptamer.

13. The aptamer conjugate of claim 12, wherein the linker is a bifunctional linker, e.g., the bifunctional linker comprises one of the following structures: 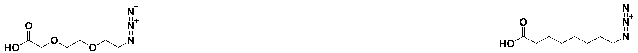 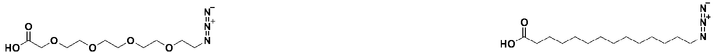 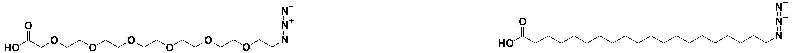 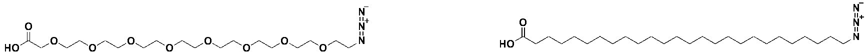 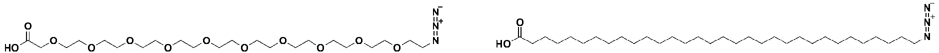 ; or the bifunctional linker comprises the following structure:

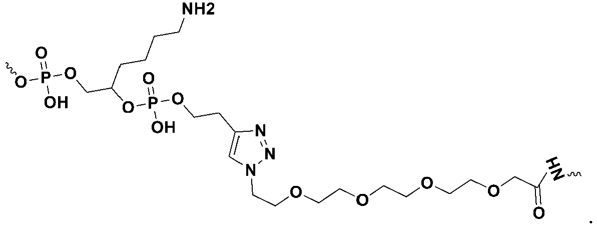

14. The aptamer conjugate of claim 13, wherein the aptamer conjugate comprises a structure of (FA/E3 ligand)-bifunctional linker-aptamer nucleotide sequence (5'-3'), wherein FA represents a fatty acid and E3 ligand represents the specific ligand of E3, wherein FA and E3 ligand are conjugated to 5' end of the aptamer nucleotide sequence through a bifunctional linker, preferably, the fatty acid is octadecanedioic acid and/or the the specific ligand of E3 is VHL.

15. The aptamer conjugate of claim 14, wherein the aptamer conjugate comprises a structure represented by the following formula:

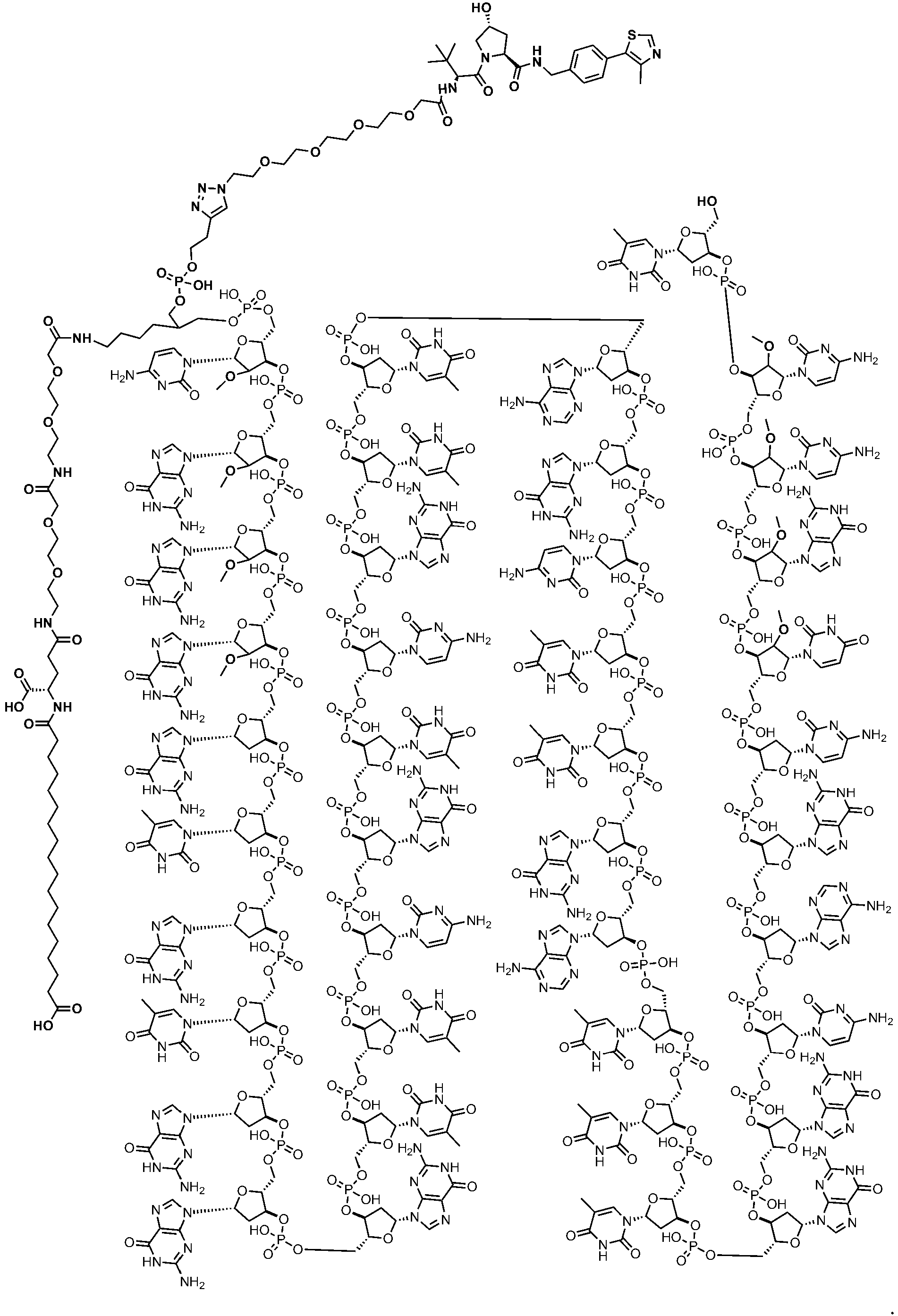

16. The aptamer conjugate of any one of claims 1 to 10, wherein the specific ligand of the asialoglycoprotein receptor (ASGPR) is N-acetylgalactosamine (GalNAc).

17. The aptamer conjugate of claim 1 to 16, wherein the specific ligand of ASGPR such as GalNAc is conjugated with the aptamer via a linker.

18. The aptamer conjugate of claim 16 or 17, wherein the aptamer conjugate comprises a structure as shown in the following formula: 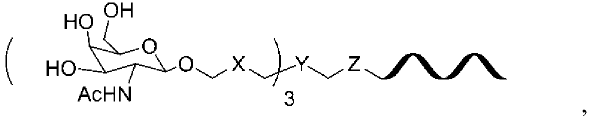 wherein
| | is selected from | preferably, |
|---|---|---|
| X | 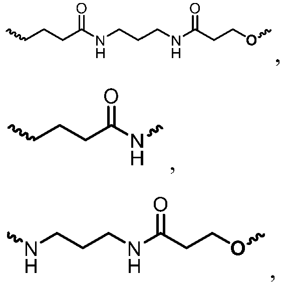 | 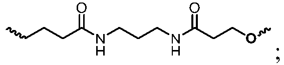 |
| Y | 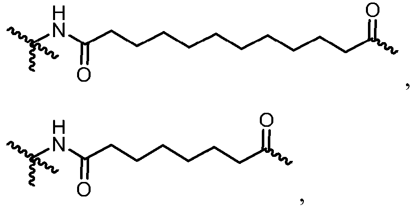 | 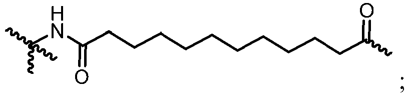 |
| Z | 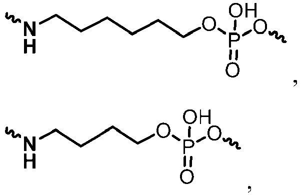 | 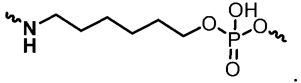 |

19. The aptamer conjugate of any one of claims 16 to 18, wherein the aptamer conjugate comprises a structure as shown in the following formula:

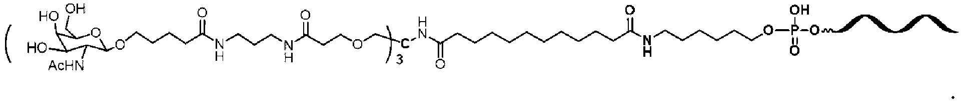

20. The aptamer conjugate of claim 1 to 19, wherein the aptamer nucleotide sequence (5'-3' direction) is
C(OMe)G(OMe)G(OMe)G(OMe)GTGTGGGTTCGTCGTTAGCTTGATTTGGCAGCU (OMe)G(OMe)C(OMe)C(OMe)-idT, wherein (OMe) represents a 2'-methoxy (2'-OMe) modification of the corresponding nucleotide and idT represents a 3' inverted deoxythymidine modification.

21. The aptamer conjugate of any one of claims 1 to 20, wherein the aptamer conjugate is used for targeted degradation of sclerostin.

22. The aptamer conjugate of claim 21, wherein the aptamer conjugate is used fortargeted degradation of sclerostin within a cell.

23. The aptamer conjugate of claim 22, wherein the cell is a cancer cell, for example, the cancer cell is a cancer cell highly expressing sclerostin or the cancer cell is a cancer cell highly expressing sclerostin and asialoglycoprotein receptor (ASGPR).

24. The aptamer conjugate of claim 23, wherein the cancer cell is a breast cancer cell (preferably triple-negative breast cancer cell) or a liver cancer cell.

25. A method of treating a sclerostin-related disease, comprising administering to a subject in need thereof a therapeutically effective amount of the aptamer conjugate according to any one of claims 1 to 20, wherein for example said subject is human.

26. The method of claim 25, wherein the sclerostin-related disease is selected from the group consisting of osteoporosis, osteopenia, osteomalacia, osteogenesis imperfecta (OI), ischemic necrosis, rheumatoid arthritis, fracture, osteoarthritis, multiple myeloma, hypophosphatemic ricket, hepatocellular carcinoma and triple-negative breast cancer.

27. A pharmaceutical composition comprising at least one of the aptamer conjugate according to any one of claims 1 to 20 and a pharmaceutically acceptable carrier or excipient.

28. Use of the aptamer conjugate according to any one of claims 1 to 20 or the pharmaceutical composition according to claim 27 in preparation of a medicament for treating a sclerostin-related disease.

29. The use of claim 28, wherein the sclerostin-related disease is selected from the group consisting of osteoporosis, osteopenia, osteomalacia, osteogenesis imperfecta (OI), ischemic bone necrosis, rheumatoid arthritis, fracture, osteoarthritis, multiple myeloma, hypophosphatemic rickets, hepatocellular carcinoma and triple negative breast cancer.
